# EUROPEAN PATENT SPECIFICATION

(11) **EP 1 214 944 B1**
(45) Date of publication and mention of the grant of the patent: **23.09.2009**
(21) Application number: 00953481.9
(22) Date of filing: 18.08.2000
(51) Int. Cl.: A61K 45/00, A61K 31/343, A61K 31/407, A61K 31/36, A61K 31/357, A61K 31/366, A61K 31/352, A61K 9/70, A61K 47/14, A61K 47/10, A61K 47/34, A61K 47/18, A61P 25/20, C07D 311/78, C07D 307/77, C07D 491/048, C07D 491/052, C07D 317/70, C07D 319/14

(54) **Composition for PERCUTANEOUS ABSORPTION with a compound having a melatonin receptor agonist activity**
Zusammensetzung für perkutane Absorption mit einem melantoninrezeptor-agonistisch wirkendem Wirkstoff
Composition pour l'ABSORPTION PERCUTANEE avec un composé ayant une activité agonistique sur le recepteur de melatonin

(30) Priority: 20.08.1999 JP 23410699
(43) Date of publication of application: 19.06.2002
(73) Proprietor: Takeda Pharmaceutical Company Limited, Osaka (JP)
(72) Inventor: SUZUKI, Yasuyuki, Nishinomiya-shi, Hyogo 663-8113 (JP); IGA, Katsumi, Suita-shi, Osaka 565-0833 (JP); MIYAMOTO, Masaomi, Takawazuka-shi, Hyogo 665-0841 (JP)
(74) Representative: Wright, Robert Gordon McRae
(86) International application number: PCT/JP2000/005525
(87) International publication number: WO 2001/013950

(56) References cited:
- EP-A- 0 916 336
- EP-A1- 0 578 620
- EP-A1- 0 879 597
- WO-A-98/17315
- WO-A1-97/32871
- JP-A- 10 182 450
- JP-A- 10 182 455
- US-A- 5 750 134
- LEE BJ ET AL: "Solubility and stability of melatonin in propylene glycol and 2-hydroxypropyl-beta-cyclodextrin vehicles" ARCHIVES OF PHARMACAL RESEARCH, NATL. FISHERIES UNIVERSITY, PUSAN, KR, vol. 20, no. 6, December 1997 (1997-12), pages 560-565, XP002108638 ISSN: 0253-6269
- BEOM-JIN L: "DEVELOPMENT AND EVALUATION OF AN ORAL CONTROLLED RELEASE AND A TRANSDERMAL DELIVERY SYSTEM FOR MELATONIN IN HUMAN SUBJECTS" DISSERTATION ABSTRACTS INTERNATIONAL, ANN ARBOR, MI, US, vol. 54, no. 2, 1 August 1993 (1993-08-01), pages 762-763, XP000570262

## Description

### TECHNICAL FIELD

The present invention relates to percutaneous absorption preparations which make it possible to continuously absorb compounds having a melatonin receptor agonism into a patient's body via a skin (contact surface) with high efficiency only during a patient's sleep (absorption decreases before the patient wakes up), and hence are effective for control of a biological rhythm, typically sleep-awake rhythm which leads a natural sleep, control of jet lag and preventive and therapeutic treatments of, for example, somnipathy.

### BACKGROUND ART

Compounds having a melatonin ML₁ receptor agonist activity bind to a melatonin ML₁ receptor on a cell membrane and express a melatonin-like action. A diurnal variation of melatonin is such that its blood concentration increases from about 8 o'clock at night, reaches the maximum concentration from about 12 o'clock to 2 o'clock in the middle of night and decreases to the initial level until about 8 o'clock in the morning. This diurnal variation decreases in accordance with aging, which is considered as one of the reasons for senile somnipathy or the like.

On the other hand, as for percutaneous absorption preparations of melatonin receptor agonist, Japanese Unexamined Patent Publications JP A 6-72874, JP A 10-182455, JP A 10-29934 and JP A 10-29933 have been currently reported.
XP 002108638 describes the solubility and stability of melatonin in propylene glycol and 2-hydroxypropyl-β-cyclodextrin vehicles.
XP 00570262 describes the development and evaluation of an oral controlled release and a transdermal delivery system for melatonin in human subjects.
W09817315 relates to the transdermal delivery of drugs and more particularly to permeation enhancers for compositions, devices and methods for enhancing the percutaneous absorption of drugs when administered to a body surface or membrane.
US5750134 discloses a bioadhesive composition that adheres suitably to a mucosal surface and is capable of delivering drugs in a sustained fashion, and a patch comprising the bioadhesive composition.
EP0916336 relates to drug delivery systems and, more specifically, to a transdermal drug delivery system and the method of use of the same, designed to deliver therapeutically effective dosages of drugs to skin cancers, lesions and infections.
JP10182455 discloses a patch used for treating insomnia and comprises a layer comprising adhesive composition containing melatonin formed on supporting material. EP0578620 relates to melatonin and/or its agonists and more specifically to pharmaceutical compositions which are very efficient in the treatment of pathologies which interfere with the circadian rhythms.
W09732871 relates to a tricyclic compound with excellent binding affinity for melatonin receptor, a process for producing and use thereof.

It is important for a patient of somnipathy that the blood concentration of melatonin peaks at 4 to 6 hours after going to bed, and hence it is also necessary for the case of the melatonin ML₁ receptor agonist to control the blood concentration so as to compensate the melatonin pattern in healthy condition. The conventional percutaneous absorption preparations of melatonin receptor agonist, however, are not satisfactory as medication for preventing or treating somnipathy or the like because its absorption efficiency is not high enough and hence it cannot provide a one-peak blood concentration passage characteristic in which the blood concentration rapidly increases after affixing before going to bed and levels off at an effective blood concentration during sleep and has decreases to an acceptable level by the time of wake-up.

The present invention provides convenient percutaneous absorption preparations of compounds having a melatonin ML₁ receptor agonist activity, that is, percutaneous absorption preparations of while-asleep-application (night affix) type for leading a normal sleep, which makes it possible that the compounds are absorbed in percutaneous manner with high efficiency during a sleep and show a melatonin-like effective blood-drug-concentration-time profile in which the blood concentration has decreased before the wakeup time in the morning and the action of the drug no longer continues at the time of wakeup.

### DISCLOSURE OF THE INVENTION

As a result of enthusiastic researches on natural sleep, the inventors of the present invention have found that percutaneous absorption preparations inventively containing a compound having a melatonin receptor agonist activity according to formula (1) and a fatty acid ester, polyhydric alcohol and a nonionic surfactant can unexpectedly penetrate the skin at a desirable speed, exhibit a blood-drug-concentration-time profile in which the blood concentration rapidly increases after administration and the effective blood concentration is kept for 6 to 12 hours in contrast to the case where the compound is orally administered, can lead a natural sleep, and hence are useful as medications for preventing or treating jet lag, somnipathy and the like as well as medications for adjusting biological rhythm.

That is, the present invention provides:
(1) A percutaneous absorption preparation containing a compound having a melatonin receptor agonist activity represented by the formula: 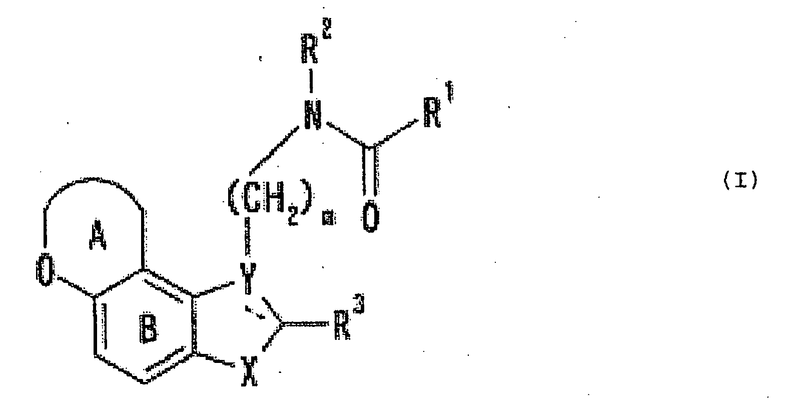 wherein, R¹ represents an optionally substituted hydrocarbon group, an optionally substituted amino group or an optionally substituted heterocyclic group;
   R² represents a hydrogen atom or an optionally substituted hydrocarbon group;
   R³ represents a hydrogen atom, an optionally substituted hydrocarbon group or an optionally substituted heterocyclic group;
   X represents CHR⁴, NR⁴, O or S in which R⁴ represents a hydrogen atom or an optionally substituted hydrocarbon group;
   Y represents C, CH or N, provided that when X is CH₂, Y is C or CH;
   -̅-̅-̅-̅-̅ represents a single bond or a double bond;
   ring A represents an optionally substituted, 5- to 7- membered oxygen-containing heterocyclic ring;
   ring B represents an optionally substituted benzene ring; and
   m represents an integer of 1 to 4;
   or a salt thereof,
   and a fatty acid ester, a polyhydric alcohol and a non-ionic surfactant;
(2) The percutaneous absorption preparation according to the above-mentioned (1), wherein the compound having a melatonin receptor agonist activity is a compound having a melatonin ML₁ receptor agonist activity;
(3) The percutaneous absorption preparation according to the above-mentioned (1), wherein the compound having a melatonin receptor agonist activity is a compound represented by the formula: 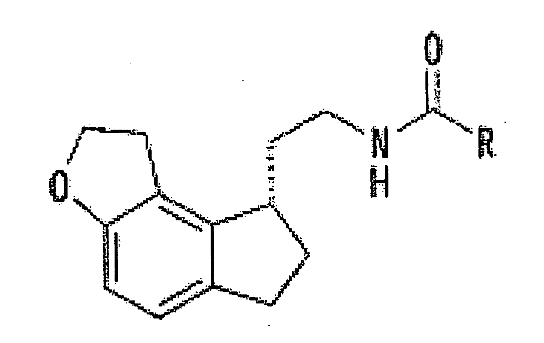 wherein, R represents a C₁₋₆ alkyl group;
(4) The percutaneous absorption preparation according to the above-mentioned (1), wherein the compound having a melatonin receptor agonist activity is (S)-N-[2-(1,6,7,8-tetrahydro-2H-indeno[5,4-b]furan-8-yl)ethyl]propionamide;
(5) The percutaneous absorption preparation according to the above-mentioned (1), wherein the compound having a melatonin receptor agonist activity is (S)-N-[2-(1,6,7,8-tetrahydro-2H-indeno[5,4-b]furan-8-yl)ethyl]acetamide.
(6) The percutaneous absorption preparation according to the above-mentioned (1), wherein the fatty acid ester is an ester of a carboxylic acid having 6 to 22 carbon atoms and an alkyl alcohol having 1 to 12 carbon atoms;
(7) The percutaneous absorption preparation according to the above-mentioned (1), wherein the fatty acid ester is isopropyl myristate, isopropyl palmitate, butyl myristate, or diethyl sebacate;
(8) The percutaneous absorption preparation according to the above-mentioned (1), wherein the fatty acid ester is isopropyl myristate;
(9) The percutaneous absorption preparation according to the above-mentioned (1), wherein the polyhydric alcohol is ethylene glycol, propylene glycol, 1,3-butylene glycol, glycerin or polyethylene glycol;
(10) The percutaneous absorption preparation according to the above-mentioned (1), wherein the polyhydric alcohol is propylene glycol;
(11) The percutaneous absorption preparation according to the above-mentioned (1), wherein the polyhydric alcohol is polyethylene glycol;
(12) The percutaneous absorption preparation according to the above-mentioned (1), wherein the polyhydric alcohol is polyethylene glycol having a molecular weight of about 200 to about 1000;
(13) The percutaneous absorption preparation according to the above-mentioned (1), wherein the nonionic surfactant is a fatty acid amide, a polyhydric alcohol fatty acid ester or a polyglycerol fatty acid ester;
(14) The percutaneous absorption preparation according to the above-mentioned (1), wherein the nonionic surfactant is a fatty acid amide;
(15) The percutaneous absorption preparation according to the above-mentioned (14), wherein the fatty acid amide is lauric diethanol amide or a.compound including the same;
(16) The percutaneous absorption preparation according to the above-mentioned (14), wherein the fatty acid amide is coconut fatty acid diethanol amide,
(17) The percutaneous absorption preparation according to the above-mentioned (1) containing (S)-N-[2-(1,6,7,8-tetrahydro-2H-indeno[5,4-b]furan-8-yl)ethyl]propionamide, isopropyl myristate, polyethylene glycol and lauric diethanol amide;
(18) The percutaneous absorption preparation according to the above-mentioned (1) containing (S)-N-[2-(1,6,7,8-tetrahydro-2H-indeno[5,4-b]furan-8-yl)ethyl]acetamide, isopropyl myristate, polyethylene glycol and lauric diethanol amide;
(19) The percutaneous absorption preparation according to the above-mentioned (1) which is a skin plaster;
(20) The percutaneous absorption preparation according to the above-mentioned (1) containing in a skin contact member, a compound having a melatonin receptor agonist activity and a fatty acid ester, a polyhydric alcohol and a nonionic surfactant;
(21) The percutaneous absorption preparation according to the above-mentioned (20) containing in a skin contact member, an about 1 to about 30% by weight of fatty acid ester with respect to a weight of the skin contact member.
(22) The percutaneous absorption preparation according to the above-mentioned (20) containing in a skin contact member, an about 1 to about 30% by weight of polyhydric alcohol with respect to a weight of the skin contact member;
(23) The percutaneous absorption preparation according to the above-mentioned (20) containing in a skin contact member, an about 1 to about 15% by weight of nonionic surfactant with respect to a weight of the skin contact member;
(24) The percutaneous absorption preparation according to the above-mentioned (20) containing in a skin contact member, an adhesive agent;
(25) The percutaneous absorption preparation according to the above-mentioned (24), wherein the adhesive agent is an acrylic adhesive agent;
(26) The percutaneous absorption preparation according to the above-mentioned (20) containing in a skin contact member, an about 0.01 to about 70% by weight of compound having a melatonin receptor agonist activity with respect to a weight of the skin contact member;
(27) The percutaneous absorption preparation according to the above-mentioned (20) containing in a skin contact member, an about 5 to about 99% by weight of adhesive agent with respect to a weight of the skin contact member;
(28) The percutaneous absorption preparation according to the above-mentioned (20), wherein a content of the compound having a melatonin receptor agonist activity per unit skin contact surface of a skin contact member is about 0.01 to about 100 mg/cm²;
(29) The percutaneous absorption preparation according to the above-mentioned (20) containing in a skin contact member, a filler;
(30) The percutaneous absorption preparation according to the above-mentioned (29), wherein the filler is silicon dioxide;
(31) The percutaneous absorption preparation according to the above-mentioned (1) which is to be affixed between about 6 hours before bedtime to just before bedtime;
(32) The percutaneous absorption preparation according to the above-mentioned (30), wherein a peak of the effective blood concentration of the compound having a melatonin receptor agonist activity appears within about 10 hours after administration; and
(33) Use of a compound having a melatonin receptor agonist activity represented by the formula: 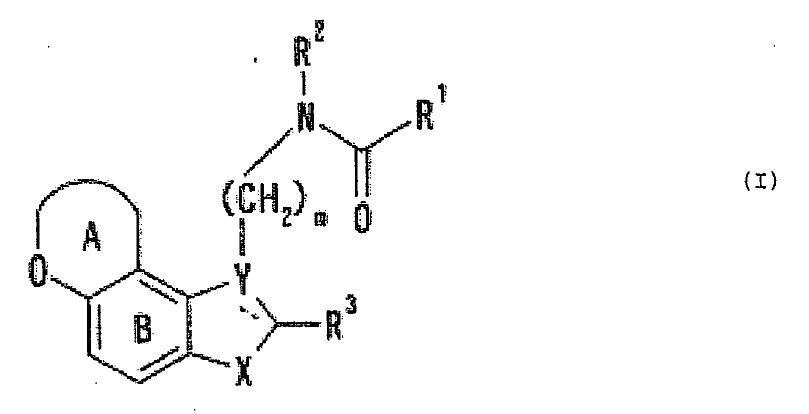 wherein, R¹ represents an optionally substituted hydrocarbon group, an optionally substituted amino group or an optionally substituted heterocyclic group;
   R² represents a hydrogen atom or an optionally substituted hydrocarbon group;
   R³ represents a hydrogen atom, an optionally substituted hydrocarbon group or an optionally substituted heterocyclic group;
   X represents CHR⁴, NR⁴, O or S in which R⁴ represents a hydrogen atom or an optionally substituted hydrocarbon group;
   Y represents C, CH or N, provided that when X is CH₂, Y is C or CH;
   -̅-̅-̅-̅-̅ represents a single bond or a double bond;
   ring A represents an optionally substituted, 5- to 7- membered oxygen-containing heterocyclic ring;
   ring B represents an optionally substituted benzene ring; and
   m represents an integer of 1 to 4;
   or a salt thereof, and a fatty acid ester, a polyhydric alcohol and a nonionic surfactant in the manufacture of a percutaneous absorption medicament for the preventive and therapeutic treatment of diseases related to melatonin.

The percutaneous absorption preparations according to the present invention are applied to the compound of formula (I) having a melatonin receptor (ML₁, ML₂, nuclear receptor, etc.) agonist activity, and among them, can preferably applied to compounds having a melatonin ML₁ receptor agonist activity.

The term "melatonin ML₁ receptor agonist activity" used in the present invention means an action of specifically binding to a melatonin ML₁ receptor which is one of the melatonin receptors on a cell membrane and proving a comparative or better effect than the case where the receptor binds to melatonin. As a result of binding to the melatonin ML₁ receptor, a sleep inducing action is derived, and this action induces a sleep which is similar to a natural sleep and causes no discomfort on the next day in contrast to the sleep action by diazepam or the like. Therefore, compounds having a melatonin ML₁ receptor agonist activity can be applied for adjustment of biological rhythms, typically sleep-awake rhythm, adjustment of a jet lag, treatment of a somnipathy and the like.

The "hydrocarbon group" in "optionally substituted hydrocarbon group" as referred to herein includes, for example, an aliphatic hydrocarbon group, a mono-cyclic saturated hydrocarbon group, an aromatic hydrocarbon group, etc.,
and this preferably has from 1 to 16 carbon atoms. Concretely, this includes, for example, an alkyl group, an alkenyl group, an alkynyl group, a cycloalkyl group, an aryl group, etc.

The "alkyl group" is, for example, preferably a lower alkyl group and generally includes C₁₋₆ alkyl groups such as methyl, ethyl, propyl, isopropyl, butyl, isobutyl, sec-butyl, tert-butyl, pentyl, hexyl, etc.

The "alkenyl group" is, for example, preferably a lower alkenyl group and generally includes C₂₋₆ alkenyl groups such as vinyl, 1-propenyl, allyl, isopropenyl, butenyl, isobutenyl, etc.

The "alkynyl group" is, for example, preferably a lower alkynyl group and generally includes C₂₋₆ alkynyl groups such as ethynyl, propargyl, 1-propynyl, etc.

The "cycloalkyl group" is, for example, preferably a lower cycloalkyl group and generally includes C₃₋₆ cycloalkyl groups such as cyclopropyl, cyclobutyl, cyclopentyl, cyclohexyl, etc.

The "aryl group" is preferably a C₆₋₁₄ aryl group, including, for example, phenyl, 1-naphthyl, 2-naphthyl, biphenylyl, 2-anthryl, etc. For example, phenyl is generally used.

The substituents for the "hydrocarbon group" of the "optionally substituted hydrocarbon group" include, for example, a halogen atom (e.g., fluorine, chlorine, bromine, iodine, etc.), a nitro group, a cyano group, a hydroxyl group, an optionally halogenated lower alkyl group (e.g., an optionally halogenated. C₁₋₆ alkyl group such as methyl, chloromethyl, difluoromethyl, trichloromethyl, trifluoromethyl, ethyl,2-bromoethyl,2,2,2- trifluoroethyl, pentafluoroethyl, propyl, 3,3,3-trifluoropropyl, isopropyl, butyl, isobutyl, sec-butyl, tert-butyl, 4,4,4-trifluorobutyl, pentyl, isopentyl, neopentyl, 5,5,5-trifluoropentyl, hexyl, 6,6,6-trifluorohexyl, etc.), a lower alkoxy group (e.g., a C₁₋₆ alkoxy group such as methoxy, ethoxy, propoxy, isopropoxy, butoxy, isobutoxy, pentyloxy, hexyloxy, etc.), an amino group, a mono-lower alkylamino group (e.g., a mono-C₁₋₆ alkylamino group such as methylamino, ethylamino, etc.), a di-lower alkylamino group (e.g., a di-C₁₋₆ lower alkylamino group such as dimethylamino, diethylamino, etc.), a carboxyl group, a lower alkylcarbonyl group (e.g., a C₁₋₆ alkyl-carbonyl group such as acetyl, propionyl, etc.), a lower alkoxycarbonyl group (e.g., a C₁₋₆ alkoxy-carbonyl group such as methoxycarbonyl, ethoxycarbonyl, propoxycarbonyl, butoxycarbonyl, etc.), a carbamoyl group, a mono-lower alkylcarbamoyl group (e.g., a mono-C₁₋₆ alkyl-carbamoyl group such as methylcarbamoyl, ethylcarbamoyl, etc.), a di-lower alkylcarbamoyl group (e.g., a di-C₁₋₆ alkyl-carbamoyl group such as dimethylcarbamoyl, diethylcarbamoyl, etc.), an arylcarbamoyl group (e.g., a C₆₋₁₀ aryl-carbamoyl group such as phenylcarbamoyl, naphthylcarbamoyl, etc.), an aryl group (e.g., a C₆₋₁₀ aryl group such as phenyl, naphthyl, etc.), an aryloxy group (e.g., a C₆₋₁₀ aryloxy group such as phenyloxy, naphthyloxy, etc.), an optionally halogenated lower alkylcarbonylamino group (e.g., an optionally halogenated C₁₋₆ alkyl-carbonylamino group such as acetylamino, trifluoroacetylamino, etc.), an oxo group, etc. The "hydrocarbon group" of the "optionally substituted hydrocarbon group" may have 1 to 5, preferably 1 to 3 substituents selected from those mentioned above, at any substitutable positions in the group. When the number of the substituents is two or more, each of the substituents may be the same or different.

The "heterocyclic group" in "optionally substituted heterocyclic group" as referred to herein includes, for example, a 5- to 14-membered (preferably, 5- to 10- membered), mono- to tri-cyclic (preferably mono- or dicyclic) heterocyclic group, each having 1 or 2 kinds, 1 to 4 (preferably 1 to 3) hetero atoms
selected from nitrogen, oxygen and sulfur, in addition to carbon atoms. Concretely, it includes, for example, a 5- membered heterocyclic group having 1 to 4 hetero atoms selected from oxygen, sulfur and nitrogen, in addition to carbon atoms, such as 2- or 3-thienyl, 2- or 3-furyl, 1-, 2- or 3-pyrrolyl, 1-, 2- or 3-pyrrolidinyl, 2-, 4- or 5-oxazolyl, 3-, 4- or 5-isoxazolyl, 2-, 4- or 5-thiazolyl, 3-, 4- or 5-isothiazolyl, 3-, 4- or 5-pyrazolyl, 2-, 3- or 4-pyrazolidinyl, 2-, 4-, or 5-imidazolyl, 1,2,3-triazolyl, 1,2,4-triazolyl, 1H- or 2H-tetrazolyl; a 6-membered heterocyclic group having 1 to 4 hetero atoms selected from oxygen, sulfur and nitrogen atoms, in addition to carbon atoms, such as 2-, 3- or 4-pyridyl, N-oxido-2-, 3- or 4-pyridyl, 2-, 4- or 5-pyrimidinyl, N-oxido-2-, 4- or 5-pyrimidinyl, thiomorpholinyl, morpholinyl, piperidino, 2-, 3- or 4-piperidyl, thiopyranyl, 1,4-oxazinyl, 1,4-thiazinyl, 1,3-thiazinyl, piperazinyl, triazinyl, 3- or 4-pyridazinyl, pyrazinyl, N-oxido-3- or 4-pyridazinyl; a di- or tri-cyclic condensed heterocyclic group having 1 to 4 hetero atoms selected from oxygen, sulfur and nitrogen atoms, in addition to carbon atoms (preferably, a group to be formed by condensing the above-mentioned 5- or 6-membered cyclic group with one or two 5- or 6-membered cyclic groups each optionally having 1 to 4 hetero atoms selected from oxygen, sulfur and nitrogen atoms, in addition to carbon atoms), such as indolyl, benzofuryl, benzothiazolyl, benzoxazolyl, benzimidazolyl, quinolyl, isoquinolyl, phthalazinyl, quinazolinyl, quinoxalinyl, indolidinyl, quinolidinyl, 1,8-naphthyridinyl, dibenzofuranyl, carbazolyl, acridinyl, phenanthridinyl, chromanyl, phenothiazinyl, phenoxazinyl, etc. Of these, preferred are 5- to 7-membered (preferably, 5- or 6-membered) heterocyclic groups each having 1 to 3 hetero atoms selected from oxygen, sulfur and nitrogen atoms, in addition to carbon atoms.

The substituents for the "heterocyclic group" of the "optionally substituted heterocyclic group" include, for example, a halogen atom (e.g., fluorine, chlorine, bromine, iodine, etc.), a lower alkyl group (e.g., a C₁₋₆ alkyl group such as methyl, ethyl, propyl, isopropyl, butyl, isobutyl, sec-butyl, tert-butyl, pentyl, hexyl, etc.), a cycloalkyl group (e.g., a C₃₋₆ cycloalkyl group such as cyclopropyl, cyclobutyl, cyclopentyl, cyclohexyl, etc.), a lower alkynyl group (e.g., a C₂₋₆ alkynyl group such as ethynyl, 1-propynyl, propargyl, etc.), a lower alkenyl group (e.g., a C₂₋₆ alkenyl group such as vinyl, allyl, isopropenyl, butenyl, isobutenyl, etc.), an aralkyl group (e.g., a C₇₋₁₁ aralkyl group such as benzyl, .alpha.-methylbenzyl, phenethyl, etc.), an aryl group (e.g., a C₆₋₁₀ aryl group such as phenyl, naphthyl, etc., preferably phenyl), a lower alkoxy group (e.g., a C₁₋₆ alkoxy group such as methoxy, ethoxy, propoxy, isopropoxy, butoxy, isobutoxy, sec-butoxy, tert-butoxy, etc.), an aryloxy group (e.g., a C₆₋₁₀ aryloxy group such as phenoxy, etc.), a lower alkanoyl group (e.g., formyl, a C₁₋₆ alkyl-carbonyl group such as acetyl, propionyl, butyryl, isobutyryl, etc.), an arylcarbonyl group (e.g., a C₆₋₁₀ aryl-carbonyl group such as benzoyl, naphthoyl, etc.), a lower alkanoyloxy group (e.g., formyloxy, a C₁₋₆ alkyl-carbonyloxy group such as acetyloxy, propionyloxy, butyryloxy, isobutyryloxy, etc.), an arylcarbonyloxy group (e.g., a C₆₋₁₀ aryl-carbonyloxy group such as benzoyloxy, naphthoyloxy, etc.), a carboxyl group, a lower alkoxycarbonyl group (e.g., a C₁₋₆ alkoxy-carbonyl group such as methoxycarbonyl, ethoxycarbonyl, propoxycarbonyl, isopropoxycarbonyl, butoxycarbonyl, isobutoxycarbonyl, tert-butoxycarbonyl, etc.), an aralkyloxycarbonyl group (e.g., a C₇₋₁₁ aralkyloxycarbonyl group such as benzyloxycarbonyl, etc.), a carbamoyl group, a mono-, di- or tri-halogeno-lower alkyl group (e.g., a mono-, di- or tri-halogeno-C₁₋₄ alkyl group such as chloromethyl, dichloromethyl, trifluoromethyl, 2,2,2-trifluoroethyl, etc.), an oxo group, an amidino group, an imino group, an amino group, a mono-lower alkylamino group (e.g., a mono-C₁₋₄ alkylamino group, such as methylamino, ethylamino, propylamino, isopropylamino, butylamino, etc.), a di-lower alkylamino group (e.g., a di-C₁₋₄ alkylamino group such as dimethylamino, diethylamino, dipropylamino, diisopropylamino, dibutylamino, methylethylamino, etc.), a 3- to 6-membered cyclic amino group optionally having 1 to 3 hetero atoms selected from oxygen, sulfur and nitrogen atoms, in addition to carbon atoms and one nitrogen atom (e.g., a 3- to 6-membered cyclic amino group such as aziridinyl, azetidinyl, pyrrolidinyl, pyrrolinyl, pyrrolyl, imidazolyl, pyrazolyl, imidazolidinyl, piperidyl, morpholinyl, dihydropyridyl, pyridyl, N-methylpiperazinyl, N-ethylpiperazinyl, etc.), an alkylenedioxy group (e.g., a C₁₋₃ alkylenedioxy group such as methylenedioxy, ethylenedioxy, etc.), a hydroxyl group, a nitro group, a cyano group, a mercapto group, a sulfo group, a sulfino group, a phosphono group, a sulfamoyl group, a monoalkylsulfamoyl group (e.g., a mono-C₁₋₆ alkylsulfamoyl group such as N-methylsulfamoyl, N-ethylsulfamoyl, N-propylsulfamoyl, N-isopropylsulfamoyl, N-butylsulfamoyl, etc.), a dialkylsulfamoyl group (e.g., a di-C₁₋₆ alkylsulfamoyl group such as N,N-dimethylsulfamoyl, N,N-diethylsulfamoyl, N,N-dipropylsulfamoyl, N,N-dibutylsulfamoyl, etc.), an alkylthio group (e.g., C₁₋₆ alkylthio group such as methylthio, ethylthio, propylthio, isopropylthio, butylthio, sec-butylthio, tert-butylthio, etc.), an arylthio group (e.g., a C₆₋₁₀ arylthio group such as phenylthio, naphthylthio, etc.), a lower alkylsulfinyl group (e.g., a C₁₋₆ alkylsulfinyl group such as methylsulfinyl, ethylsulfinyl, propylsulfinyl, butylsulfinyl, etc.), an arylsulfinyl group (e.g., a C₆₋₁₀ arylsulfinyl group such as phenylsulfinyl, naphthylsulfinyl, etc.), a lower alkylsulfonyl group (e.g., a C₁₋₆ alkylsulfonyl group such as methylsulfonyl, ethylsulfonyl, propylsulfonyl, butylsulfonyl, etc.), an arylsulfonyl group (e.g., a C₆₋₁₀ arylsulfonyl group such as phenylsulfonyl, naphthylsulfonyl, etc.), etc.

The "heterocyclic group" of the "optionally substituted heterocyclic group" may have 1 to 5, preferably 1 to 3 substituents selected from those mentioned above, at any substitutable positions in the group. In the case that the group has two or more substituents, these substituents may be the same or different.

The "optionally substituted amino group" as referred to herein includes amino groups each optionally having one or two substituents of, for example, the above-mentioned "optionally substituted hydrocarbon groups". Preferred substituents for the above "amino group" include, for example, an optionally substituted C₁₋₆ alkyl group and an optionally substituted C₆₋₁₀ aryl group. The substituents which the "C₁₋₆ alkyl group" or the "C₆₋₁₀ aryl group" may optionally have are, for example, the same ones as the above-mentioned "hydrocarbon group" may optionally have.

The "lower alkyl group" for "optionally substituted lower alkyl group" as referred to herein includes, for example, a C₁₋₆ alkyl group such as methyl, ethyl, propyl, isopropyl, butyl, isobutyl, sec-butyl and tert-butyl. The lower alkyl group may optionally have 1 to 3 substituents, such as the same ones as the above-mentioned "hydrocarbon group" may optionally have.

The "lower alkoxy group" in "optionally substituted lower alkoxy group" as referred to herein includes, for example, a C₁₋₆ alkoxy group such as methoxy, ethoxy, propoxy, isopropoxy, butoxy, isobutoxy, sec-butoxy and tert-butoxy. The lower alkoxy group may optionally have 1 to 3 substituents, such as the same ones as the above-mentioned "hydrocarbon group" may optionally have.

The "optionally substituted benzene ring" as referred to herein includes, for example, a benzene ring which may optionally have one or two substituents selected from, a halogen atom (e.g., fluorine, chlorine, bromine, iodine, etc.), an optionally substituted hydrocarbon group, an optionally substituted amino group, an amide group (e.g., a C₁₋₃ acylamino group such as formamide, acetamide, etc.), an optionally substituted lower alkoxy group and a lower alkylenedioxy group (e.g., a C₁₋₃ alkylenedioxy group such as methylenedioxy, ethylenedioxy, etc.), at any substitutable positions in the ring.

For these "optionally substituted hydrocarbon group", "optionally substituted amino group" and "optionally substituted lower alkoxy group", the same ones as those described in detail hereinabove are referred to. In the case that these "hydrocarbon group", "amino group" and "lower alkoxy group" each have two or more substituents, these substituents may be the same or different.

The "optionally substituted benzene ring" is preferably a benzene ring optionally substituted by 1 or 2 substituents selected from a halogen atom (e.g., fluorine, chlorine, etc.), a C₁₋₆ alkyl group (e.g., methyl, ethyl, etc.) and a mono-C₁₋₆ alkylamino group.

In the above-mentioned formulae, R¹ represents an optionally substituted hydrocarbon group, an optionally substituted amino group or an optionally substituted heterocyclic group.

The "hydrocarbon group" of the "optionally substituted hydrocarbon group" represented by R¹ is preferably, for example, an alkyl group (e.g., a C₁₋₆ alkyl group such as methyl, ethyl, propyl, isopropyl, etc.), an alkenyl group (e.g., C₂₋₆ alkenyl group such as vinyl, etc.), an alkynyl group (e.g., a C₂₋₆ alkynyl group such as ethynyl), a cycloalkyl group (e.g., a C₃₋₆ cycloalkyl group such as cyclopropyl, cyclobutyl, cyclopentyl, cyclohexyl, etc.), or an aryl group (e.g., a C₆₋₁₄ aryl group such as phenyl, etc.), especially preferably an alkyl group (e.g., a C₁₋₆ alkyl group such as methyl, etc.) or a cycloalkyl group (e.g., a C₃₋₆ cyclopropyl group such as cyclopropyl, etc.). These "alkyl group", "alkenyl group", "alkynyl group", "cycloalkyl group" and "aryl group" each may have 1 to 5, preferably 1 to 3 substituents, such as the same ones as the above-mentioned "hydrocarbon group" may optionally have, preferably halogen atoms such as fluorines.

Preferred substituents for the "optionally substituted amino group" represented by R¹, are one or two substituents selected from, for example, an optionally substituted lower alkyl group and an optionally substituted aryl group, more preferably one substituent of an optionally substituted lower alkyl group. The "lower alkyl group" includes, for example, a C₁₋₆ alkyl group such as methyl, ethyl, propyl, isopropyl, butyl, isobutyl, sec-butyl and tert-butyl. The "lower alkyl group" may optionally have 1 to 3 substituents, such as the same ones as the above-mentioned "hydrocarbon group" may optionally have. The "aryl group" includes, for example, a C₆₋₁₀ aryl group such as phenyl, etc. The "aryl group" may optionally have 1 to 5, preferably 1 to 3 substituents, such as the same ones as the above-mentioned "hydrocarbon group" may optionally have, preferably those selected from, for example, a halogen atom such as fluorine and chlorine and a C₁₋₆ alkoxy group such as methoxy and ethoxy. The "optionally substituted amino group" includes, for example, a phenylamino group substituted by, 1 to 3 lower alkoxy groups (e.g., C₁₋₄ alkoxy groups such as methoxy, etc.) or a monoalkylamino group substituted by one lower alkyl group (e.g., a C₁₋₄ alkyl group such as methyl, ethyl, propyl, butyl, tert-butyl, etc.)

The "heterocyclic group" of the "optionally substituted heterocyclic group" represented by R¹ is, for example, preferably a 5- or 6-membered heterocyclic group having 1 to 3 hetero atoms selected from nitrogen, oxygen and sulfur atoms in addition to carbon atoms. Concretely, it includes, for example, 1-, 2- or 3-pyrrolidinyl, 2- or 4-imidazolinyl, 2-, 3- or 4-pyrazolidinyl, piperidino, 2-, 3- or 4-piperidyl, 1- or 2-piperazinyl, morpholinyl, 2- or 3-thienyl, 2-, 3- or 4-pyridyl, 2- or 3-furyl, pyrazinyl, 2-pyrimidinyl, 3-pyrrolyl, 3-pyridazinyl, 3-isothiazolyl and 3-isoxazolyl. Especially preferably, it is a 6-membered nitrogen-containing heterocyclic group (e.g., pyridyl, etc.).

Preferred substituents for the "optionally substituted heterocyclic group" represented by R¹ include, for example, a halogen atom (e.g., chlorine, fluorine, etc.), a C₁₋₆ alkyl group (e.g., methyl, ethyl, etc.), a C₁₋₆ alkoxy group (e.g., methoxy, ethoxy, etc.) and an aralkyloxycarbonyl group (e.g., a C₇₋₁₂ aralkyloxy-carbonyl group such as benzyloxycarbonyl, etc.).

R¹ is, for example, preferably (i) an optionally substituted lower alkyl group, (ii) an optionally substituted lower cycloalkyl group, (iii) an optionally substituted lower alkenyl group, (iv) an optionally substituted aryl group, (v) an optionally substituted mono- or di-lower alkylamino group, (vi) an optionally substituted arylamino group or (vii) an optionally substituted 5- or 6-membered nitrogen-containing heterocyclic group.

The "lower alkyl group" is preferably a C₁₋₆ alkyl group such as methyl, ethyl, propyl, isopropyl, butyl, pentyl and hexyl. The "lower cycloalkyl group" is preferably a C₃₋₆ cycloalkyl group such as cyclopropyl, cyclobutyl, cyclopentyl and cyclohexyl. The "lower alkenyl group" is preferably a C₂₋₆ alkenyl group such as vinyl, 1-propenyl and butenyl. The "aryl group" is preferably a C₆₋₁₀ aryl group such as phenyl, 1-naphthyl and 2-naphthyl. The "lower alkylamino group" is preferably a mono- or di-C₁₋₆ alkylamino group such as methylamino, ethylamino, propylamino, isopropylamino, butylamino, tert-butylamino, dimethylamino, diethylamino and methylethylamino. The "arylamino group" is preferably a C₆₋₁₀ arylamino group such as phenylamino. The "5- or 6-membered nitrogen-containing heterocyclic group" is, for example, preferably 2-, 3- or 4-pyridyl or the like. These groups may each optionally have 1 to 5 substituents such as those referred to the mentioned-above "hydrocarbon group" may optionally have.

More preferably, R¹ is (i) a C₁₋₆ alkyl group optionally substituted by 1 to 4 substituents selected from a halogen atom and a C₁₋₆ alkoxy group, (ii) a C₃₋₆ cycloalkyl group, (iii) a C₂₋₆ alkenyl group, (iv) a C₆₋₁₀ aryl group optionally substituted by 1 to 4 substituents selected from a C₁₋₆ alkoxy group, a nitro group, a halogeno-C₁₋₆ alkyl-carbonylamino group and a halogen atom, (v) a mono- or di-C₁₋₆ alkylamino group, (vi) a C₆₋₁₀ arylamino group optionally substituted by one to three C₁₋₆ alkoxy groups, or (vii) a 6-membered nitrogen-containing heterocyclic group optionally substituted by one or two C₇₋₁₁ aralkyloxycarbonyl groups. Even more preferably, R¹ is an optionally halogenated C₁₋₆ alkyl group (e.g., methyl, chloromethyl, difluoromethyl, trichloromethyl, trifluoromethyl, ethyl, 2-bromoethyl, 2,2,2-trifluoroethyl, pentafluoroethyl, propyl, 3,3,3-trifluoropropyl, isopropyl, butyl, isobutyl, sec-butyl, tert-butyl, 4,4,4-trifluorobutyl, pentyl, isopentyl, neopentyl, 5,5,5-trifluoropentyl, hexyl, 6,6,6-trifluorohexyl, etc.), a C₃₋₆ cycloalkyl group (e.g., cyclopropyl, cyclobutyl, cyclopentyl, cyclohexyl, etc.) or a mono-C₁₋₆ alkylamino group (e.g., methylamino, ethylamino, propylamino, isopropylamino, butylamino, tert-butylamino, etc.) Among others, R¹ is preferably an optionally halogenated C₁₋₆ alkyl group or a mono-C₁₋₆ alkylamino group, especially an optionally halogenated C₁₋₆ alkyl, in particular C₁₋₃ alkyl group (e.g., methyl, ethyl, propyl, etc.).

In the above-mentioned formulae, R² represents a hydrogen atom or an optionally substituted hydrocarbon group.

R² is preferably a hydrogen atom or an optionally substituted lower (C₁₋₆) alkyl group, more preferably a hydrogen atom or a lower (C₁₋₆) alkyl group, even more preferably a hydrogen atom.

In the above-mentioned formulae, R³ represents a hydrogen atom, an optionally substituted hydrocarbon group or optionally substituted heterocyclic group.

The "hydrocarbon group" of the "optionally substituted hydrocarbon group" represented by R³ is preferably, for example, an alkyl group (e.g., a C₁₋₆ alkyl group such as methyl, ethyl, propyl, isopropyl, etc.), an alkenyl group (e.g., a C₂₋₆ alkenyl group such as vinyl, etc.), an alkynyl group (e.g., a C₂₋₆ alkynyl group such as ethynyl, etc.), a cycloalkyl group (e.g., a C₃₋₆ cycloalkyl group such as cyclopropyl, cyclobutyl, cyclopentyl, cyclohexyl, etc.) or an aryl group (e.g., a C₆₋₁₄ aryl group such as phenyl, etc.). It is more preferably an alkyl group (e.g., a C₁₋₆ alkyl group such as methyl, etc.) or an aryl group (e.g., a C₆₋₁₄ aryl groups such as phenyl, etc.). These "alkyl group", "alkenyl group", "alkynyl group", "cycloalkyl group" and "aryl group" each may optionally have 1 to 5, preferably 1 to 3 substituents such as the same ones the mentioned-above "hydrocarbon group" may optionally have (e.g., halogen atoms such as fluorines, etc.).

The "heterocyclic group" of the "optionally substituted heterocyclic group" represented by R³ is preferably a 5- or 6-membered heterocyclic group having 1 to 3 hetero atoms selected from nitrogen, oxygen and sulfur atoms, in addition to carbon atoms. Concretely, it includes, for example, 1-, 2- or 3-pyrrolidinyl, 2- or 4-imidazolinyl, 2-, 3- or 4-pyrazolidinyl, piperidino, 2-, 3- or 4-piperidyl, 1- or 2-piperazinyl, morpholinyl, 2- or 3-thienyl, 2-, 3- or 4-pyridyl, 2- or 3-furyl, pyrazinyl, 2-pyrimidinyl, 3-pyrrolyl, 3-pyridazinyl, 3-isothiazolyl, 3-isoxazolyl, etc. More preferred is a 6-membered nitrogen-containing heterocyclic group (e.g., pyridyl, etc.).

Preferred substituents for the "optionally substituted heterocyclic group" represented by R³ include, for example, a halogen atom (e.g., chlorine, fluorine, etc.), a C₁₋₆ alkyl group (e.g., methyl, ethyl, etc.), a C₁₋₆ alkoxy group (e.g., methoxy, ethoxy, etc.), an aralkyloxycarbonyl group (e.g., a C₇₋₁₂ aralkyloxy-carbonyl group such as benzyloxycarbonyl, etc.), an amino group, a mono-C₁₋₆ alkylamino group (e.g., methylamino, ethylamino, etc.) a di-C₁₋₆ alkylamino group (e.g., dimethylamino, diethylamino, etc.) etc.

R³ is, for example, preferably (i) a hydrogen atom, (ii) an optionally substituted lower alkyl group, (iii) an optionally substituted aryl group, (iv) an optionally substituted 5- or 6-membered heterocyclic group, etc., more preferably, for example, (i) a hydrogen atom, (ii) a lower alkyl group, (iii) an optionally substituted C₆₋₁₀ aryl group, (iv) an optionally substituted 6-membered nitrogen-containing heterocyclic group. The above substituents include, for example, a halogen atom, a C₁₋₆ alkyl group, a C₁₋₆ alkoxy group, an amino group, a mono-C₁₋₆ alkylamino group, a di-C₁₋₆ alkylamino group, etc. More preferably, R³ is, for example, a hydrogen atom, a phenyl group and a 2-, 3- or 4-pyridyl group, especially preferably is a hydrogen atom.

In the above-mentioned formulae, X represents CHR⁴, NR⁴, O or S in which R⁴ represents a hydrogen atom or an optionally substituted hydrocarbon group.

R⁴ is preferably a hydrogen atom or an optionally substituted lower (C₁₋₆) alkyl group, respectively. More preferred is a hydrogen atom.

X is preferably CHR⁴ in which R⁴ is as defined above, O or S. Or, X is preferably CHR⁴ or NR⁴ in which R⁴ is as defined above.

In the above formulae, Y represents C, CH or N. Y is preferably C or CH.

In the above-mentioned formulae, ring A represents an optionally substituted, 5- to 7-membered oxygen-containing heterocyclic ring.

The "5- to 7-membered oxygen-containing heterocyclic ring" includes 5- to 7-membered (preferably 5- or 6- membered) heterocyclic rings optionally having 1 or 2 kinds, 1 to 3 hetero atoms selected from nitrogen, oxygen and sulfur atoms, in addition to carbon atoms and an oxygen atom. The above-mentioned heterocyclic ring is preferably a ring represented by the formula: 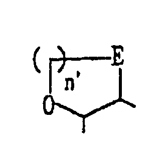 wherein E represents (i) CH₂CH₂, (ii) CH=CH, (iii) CH₂O, (iv) OCH₂, (v) CH₂S(O)_{q'} wherein q' represents an integer of 0 to 2, (vi) S(O)_{q'}CH₂ wherein q' is as defined above, (vii) CH₂NH, (viii) NHCH₂, (ix) N=N, (x) CH=N, (xi) N=CH or (xii) CONH; and n' represents an integer of 0 to 2.

E is preferably (i) CH₂CH₂, (ii) CH=CH, (iii) CH₂O, (iv) OCH₂, (v) CH₂NH, (vi) NHCH₂, (vii) N=N, (viii) CH=N or (ix) N=CH, especially preferably (i) CH₂CH₂ or (ii) CH=CH.

Concretely, the above ring includes, for example, a 5- membered oxygen-containing heterocyclic ring such as 2,3-dihydrofuran, furan, 1,3-dioxole, oxazoline, isoxazole, 1,2,3-oxadiazole and oxazole and a 6-membered oxygen-containing heterocyclic ring such as 2H-3,4-dihydropyran, 2H-pyran, 2,3-dehydro-1,4-dioxane and 2,3-dehydromorpholine.

More preferably, the above ring is a ring represented by the formula: 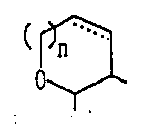 wherein n is as defined above.

Concretely, 2,3-dihydrofuran, furan, 2H-3,4-dihydropyran and 2H-pyran are preferred.

Substituents which ring A may optionally have, include, for example, a halogen atom (e.g., fluorine, chlorine, bromine, iodine, etc.), an optionally substituted lower alkyl group, an optionally substituted cycloalkyl group, an optionally substituted lower alkynyl group, an optionally substituted lower alkenyl group, an optionally substituted aryl group, a lower alkoxy group (e.g., a C₁₋₆ alkoxy group such as methoxy, ethoxy, propoxy, isopropoxy, butoxy, isobutoxy, sec-butoxy, tert-butoxy, etc.), an aryloxy group (e.g., a C₆₋₁₀ aryloxy group such as phenoxy, etc.), a lower alkanoyl group (e.g., formyl, a C₁₋₆ alkyl-carbonyl group such as acetyl, propionyl, butyryl, isobutyryl, etc.), an arylcarbonyl group (e.g., a C₆₋₁₀ aryl-carbonyl group such as benzoyl, naphthoyl, etc.), a lower alkanoyloxy group (e.g., formyloxy, a C₁₋₆ alkyl-carbonyloxy group such as acetyloxy, propionyloxy, butyryloxy, isobutyryloxy, etc.), an arylcarbonyloxy group (e.g., a C₆₋₁₀ aryl-carbonyloxy group such as benzoyloxy, naphthoyloxy, etc.), a carboxyl group, a lower alkoxycarbonyl group (e.g., a C₁₋₆ alkoxy-carbonyl group such as methoxycarbonyl, ethoxycarbonyl, propoxycarbonyl, isopropoxycarbonyl, butoxycarbonyl, isobutoxycarbonyl, tert-butoxycarbonyl, etc.), an aralkyloxy group (e.g., a C₇₋₁₁ aralkyloxy-carbonyl group such as benzyloxycarbonyl, etc.), a carbamoyl group, a thiocarbamoyl group, a mono-, di- or tri-halogeno-lower alkyl group (e.g., a mono-, di- or tri-halogeno-C₁₋₄ alkyl group such as chloromethyl, dichloromethyl, trifluoromethyl, 2,2,2-trifluoroethyl, etc.), an oxo group, an amidino group, an imino group, an amino group, a mono-lower alkylamino group (e.g., a mono-C₁₋₄ alkylamino group such as methylamino, ethylamino, propylamino, isopropylamino, butylamino, etc.), a di-lower alkylamino group (e.g., a di-C₁₋₄ alkylamino group such as dimethylamino, diethylamino, dipropylamino, diisopropylamino, dibutylamino, methylethylamino, etc.), a 3- to 6-membered cyclic amino group optionally having 1 to 3 hetero atoms selected from, for example, oxygen, sulfur and nitrogen atoms, in addition to carbon atoms and one nitrogen atom (e.g., a 3- to 6- membered cyclic amino group such as aziridinyl, azetidinyl, pyrrolidinyl, pyrrolinyl, pyrrolyl, imidazolyl, pyrazolyl, imidazolidinyl, piperidyl, morpholinyl, dihydropyridyl, pyridyl, N-methylpiperazinyl, N-ethylpiperazinyl, etc.), an alkylenedioxy group (e.g., a C₁₋₃ alkylenedioxy group such as methylenedioxy, ethylenedioxy, etc.), a hydroxyl group, a nitro group, a cyano group, a mercapto group, a sulfo group, a sulfino group, a phosphono group, a sulfamoyl group, a monoalkylsulfamoyl group (e.g., a mono-C₁₋₆ alkylsulfamoyl group such as N-methylsulfamoyl, N-ethylsulfamoyl, N-propylsulfamoyl, N-isopropylsulfamoyl, N-butylsulfamoyl, etc.), a dialkylsulfamoyl group (e.g., a di-C₁₋₆ alkylsulfamoyl group such as N,N-dimethylsulfamoyl, N,N-diethylsulfamoyl, N,N-dipropylsulfamoyl, N,N-dibutylsulfamoyl, etc.), an alkylthio group (e.g., a C₁₋₆ alkylthio group such as methylthio, ethylthio, propylthio, isopropylthio, butylthio, sec-butylthio, tert-butylthio, etc.), an arylthio group (e.g., a C₆₋₁₀ arylthio group such as phenylthio, naphthylthio, etc.), a lower alkylsulfinyl group (e.g., a C₁₋₆ alkylsulfinyl group such as methylsulfinyl, ethylsulfinyl, propylsulfinyl, butylsulfinyl, etc.), an arylsulfinyl group
(e.g., a C₆₋₁₀ arylsulfinyl group such as phenylsulfinyl, naphthylsulfinyl, etc.), a lower alkylsulfonyl group (e.g., a C₁₋₆ alkylsulfonyl group such as methylsulfonyl, ethylsulfonyl, propylsulfonyl, butylsulfonyl, etc.), an arylsulfonyl group (e.g., a C₆₋₁₀ arylsulfonyl group such as phenylsulfonyl, naphthylsulfonyl, etc.), etc.

The above "lower alkyl group", "lower alkenyl group", "lower alkynyl group", "lower cycloalkyl group" and "aryl group" each may optionally have the same ones as the above-mentioned 1 to 5, preferably 1 to 3 substituents such as those "hydrocarbon group" may optionally have.

Preferred substituents which ring A may optionally have, include, for example, a halogen atom, an optionally substituted C₁₋₆ alkyl group, an optionally substituted C₁₋₆ alkoxy group, a hydroxyl group, a nitro group, a cyano group, an optionally substituted amino group and an oxo group. For the substituents in these "optionally substituted C₁₋₆ alkyl group", "optionally substituted C₁₋₆ alkoxy group" and "optionally substituted amino group", for example, referred to are the substituents which mentioned-above "hydrocarbon group" may optionally have.

Ring A may have 1 to 4, preferably 1 or 2 substituents selected from those mentioned above at any substitutable positions, depending on the number of the carbon atoms constituting them. When the ring has two or more substituents, these substituents may be the same or different.

Ring A is, for example; 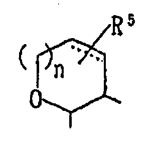 wherein n is as defined above; and R⁵ represents a hydrogen atom or 1 or 2 substituents selected from the "preferred substituents for ring A mentioned hereinabove. Among them, preferred is the one wherein R⁵ is a hydrogen atom or an optionally substituted lower (C₁₋₆) alkyl. More preferred is the one wherein R⁵ is a hydrogen atom, which indicates unsubstituted ring A.

In the above-mentioned formulae, ring B represents an optionally substituted benzene ring.

The substituents which ring B may optionally have, include, for example, the "substituents" mentioned hereinabove for the "optionally substituted benzene ring". Among others, the substituents on ring B are preferably a halogen atom or an optionally substituted lower (C₁₋₆) alkyl group, more preferably a halogen atom or a lower (C₁₋₆) alkyl group (especially, methyl). As for the substituents for the "optionally substituted lower (C₁₋₆) alkyl group", for example, referred to are the same ones as the mentioned-above "hydrocarbon group" may optionally have.

Ring B may have one or two, preferably one substituent selected from those mentioned hereinabove, at any substitutable position. When ring B has two substituents, they may be the same or different.

For example, ring B is preferably 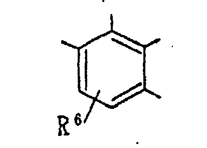 wherein R⁶ represents a hydrogen atom, a halogen atom, an optionally substituted lower (C₁₋₆) alkyl group or an optionally substituted lower (C₁₋₆) alkoxy group. R⁶ is preferably a hydrogen atom, a halogen atom or a lower (C₁₋₆) alkyl group (especially, methyl). More preferably, R⁶ is a hydrogen atom.

In the above-mentioned formulae, m represents an integer of 1 to 4. Preferably, m is an integer of 1 to 3. More preferred is 2 or 3. Especially 2 is preferable.

In the above-mentioned formulae, n represents an integer of 0 to 2. Preferably, n is an integer of 0 or 1. Especially 0 is preferable.

Examples of 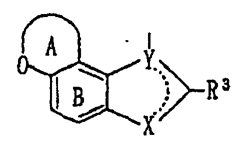 include 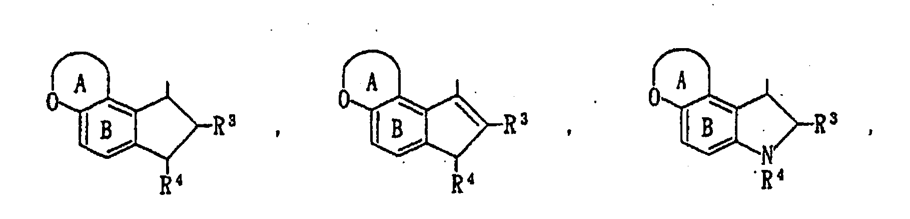 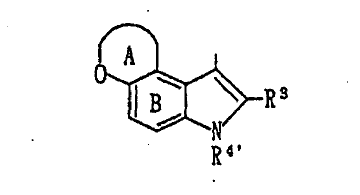 wherein R^{4'} represents an optionally substituted hydrocarbon group and the other symbols are as defined above.

R^{4'} is preferably an optionally substituted lower (C₁₋₃) alkyl group.

Preferred examples of 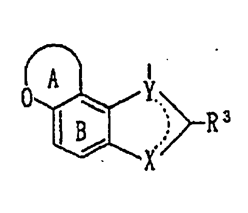 include 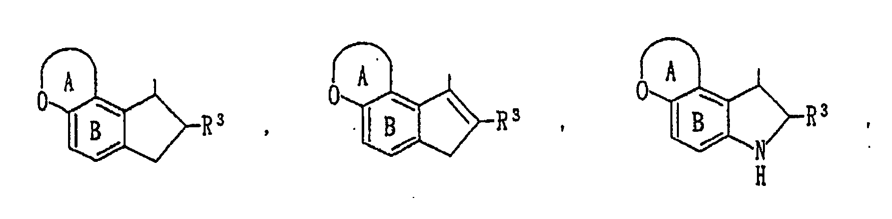 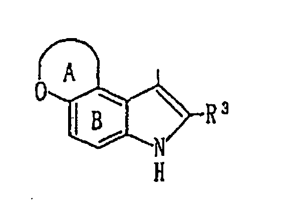 wherein are symbols are as defined above. Among them, preferred are 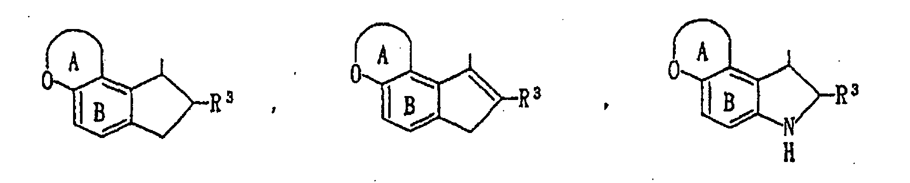 wherein the symbols are as defined above. Further preferred are 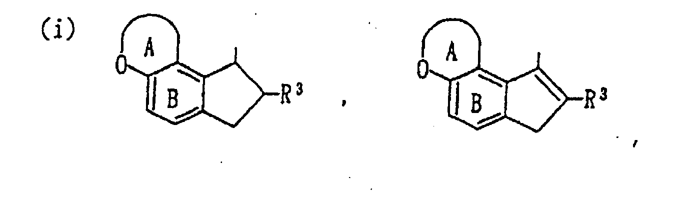 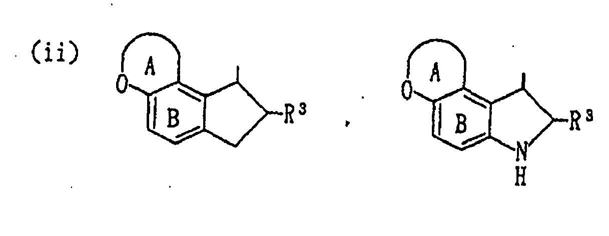 or 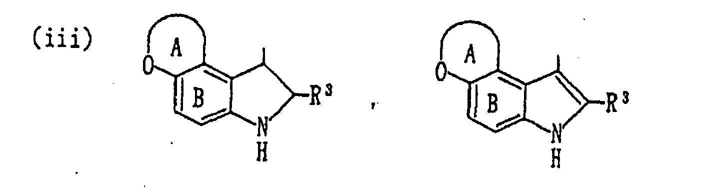 wherein the symbols are as defined above. More preferred are 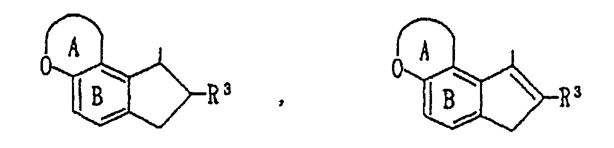 wherein the symbols are as defined above. Especially preferred is 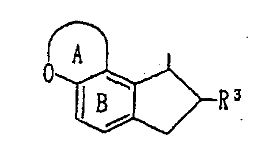 wherein the symbols are as defined above.

Example of the compound (I) include compounds having the following structural formulae. 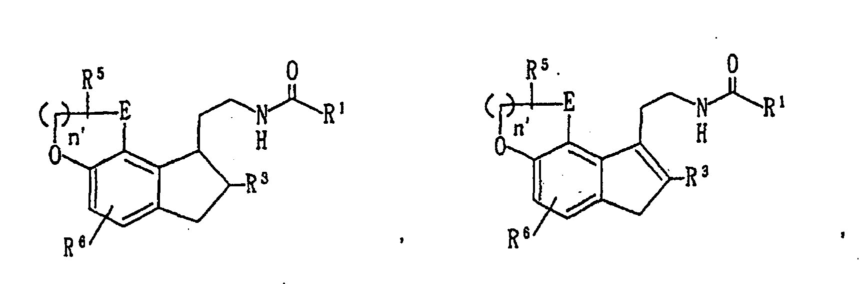 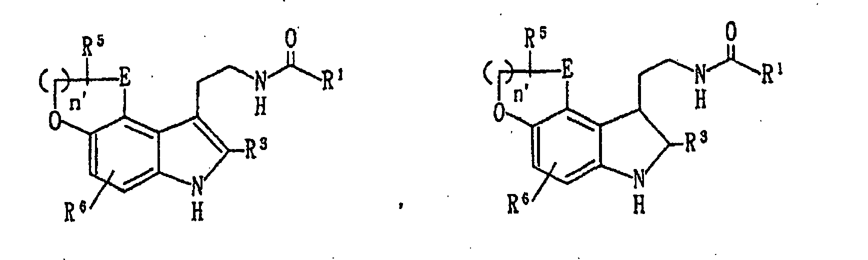 wherein the symbols are as defined above.

Preferred examples of the compound (I) include, for example, compounds of the following formulae: 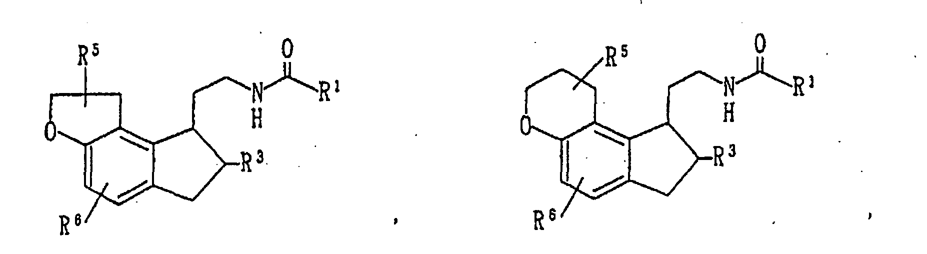 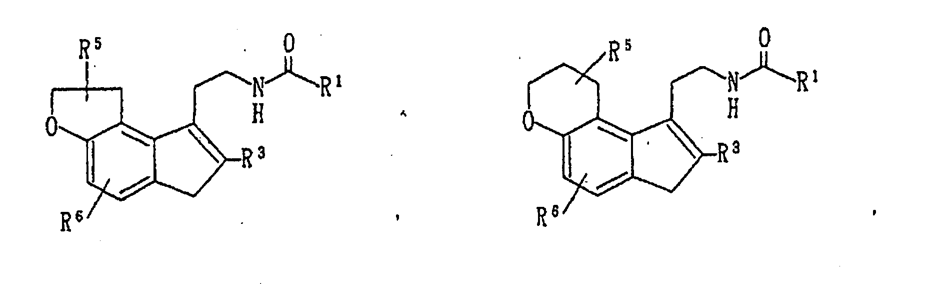 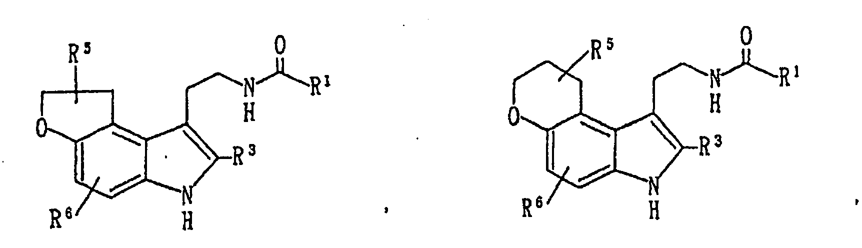 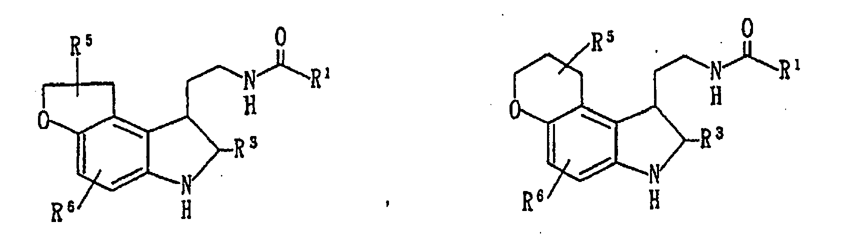 wherein the symbols are as defined above.

Also preferred examples of the compound (I) are the compound of the formula (I) wherein;
R¹ is (i) an optionally substituted lower alkyl group, (ii) an optionally substituted lower cycloalkyl group, (iii) an optionally substituted lower alkenyl group, (iv) an optionally substituted aryl group, (v) an optionally substituted mono- or di-lower alkylamino group, (vi) an optionally substituted arylamino group or (vii) an optionally substituted, 5- or 6-membered nitrogen-containing heterocyclic group;
R² is a hydrogen atom or an optionally substituted lower (C₁₋₆) alkyl group;
R³ is (i) a hydrogen atom, (ii) an optionally substituted lower alkyl group or (iii) an optionally substituted aryl group; X is CHR⁴ or NR⁴ wherein R⁴ is a hydrogen atom or a lower (C₁₋₆) alkyl group optionally substituted by an oxo group;
Y is C, CH or N, provided that when X is CH₂, Y is C or CH;
-̅-̅-̅-̅-̅ is a single bond or a double bond;
ring A is an optionally substituted, 5- to 7-membered oxygen-containing heterocyclic ring;
ring B is an optionally substituted benzene ring; and
m is 1 or 2.

More preferred is the compound wherein:
R¹ is (i) a C₁₋₆ alkyl group optionally substituted by 1 to 4 substituents selected from the group consisting of a halogen and a C₁₋₆ alkoxy group, (ii) a C₃₋₆ cycloalkyl group, (iii) a C₂₋₆ alkenyl group, (iv) a C₆₋₁₀ aryl group optionally substituted by 1 to 4 substituents selected from the group consisting of a C₁₋₆ alkoxy group, a nitro group, a halogeno-C₁₋₆ alkyl-carbonylamino group and a halogen, (v) a mono- or di-C₁₋₆ alkylamino group, (vi) a C₆₋₁₀ arylamino group optionally substituted by 1 to 3 C₁₋₆ alkoxy groups or (vii) a 6-membered nitrogen-containing heterocyclic group optionally substituted by one or two C₇₋₁₁ aralkyloxy-carbonyl groups;
R² is a hydrogen atom or a lower (C₁₋₆) alkyl group;
R³ is (i) a hydrogen atom, (ii) a lower (C₁₋₆) alkyl group or (iii) a C₆₋₁₄ aryl group;
X is CHR⁴ or NR⁴ wherein R⁴ is a hydrogen atom or a lower (C₁₋₆) alkyl group optionally substituted by an oxo group;
Y is C, CH or N, provided that when X is CH₂, Y is C or CH;
-̅-̅-̅-̅-̅ is a single bond or a double bond;
ring A is 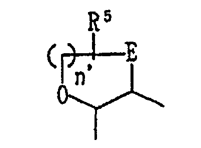 wherein the symbols are as defined above;
ring B is 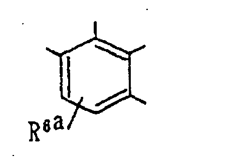 wherein R⁶a represents a hydrogen atom, a halogen atom or a lower (C₁₋₆) alkyl group; and
m is 1 or 2.

Preferred among them is the compound represented by the formula: 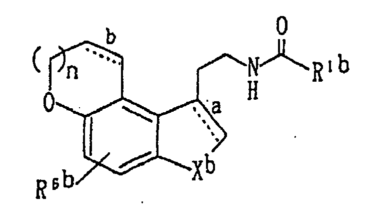 wherein R^{1b} represents a C₁₋₆ alkyl group, R^{6b} represents a 7hydrogen atom or a halogen atom, n represents 0 or 1, 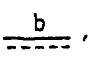
represents a single bond or a double bond, 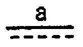 represents a single bond or a double bond when X^{b} is CH₂, and 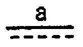 represents a single bond when X^{b} is NH; and a salt thereof.

Preferred among them is also the compound by the formula: 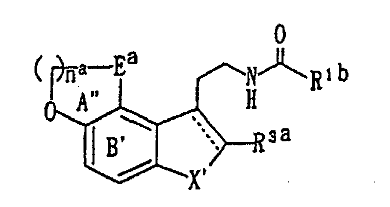 wherein R^{1b} is C₁₋₆ alkyl,
X' is CH₂, NH or NCHO,
R^{3a} is a hydrogen atom or a phenyl group,
-̅-̅-̅-̅-̅ is a single bond or double bond,
E^{a} is CH₂CH₂, CH=CH, CH₂O, CH=N, CONH or CH₂NH,
n^{a} is 0 or 1,
ring A" is a 5- or 6-membered oxygen-containing heterocyclic ring which may be substituted by 1 or 2 C₁₋₆ alkyl optionally substituted by a hydroxy, and ring B' is a benzene ring which may be substituted by a halogen); and a salt thereof. Among them, the compound wherein -̅-̅-̅-̅-̅ is a single bond ora double bond when X' is CH₂ or NCHO, and
-̅-̅-̅-̅-̅ is a single bond when X' is NH is also preferred.

Preferable examples of the compound (I) include, N-[2-(1,6,7,8-tetrahydro-2H-indeno[5,4-b]furan-8-yl)ethyl]acetamide, N-[2-(1,6,7,8-tetrahydro-2H-indeno[5,4-b]furan-8-yl)ethyl]butylamide, N-[2-(1,6,7,8-tetrahydro-2H-indeno[5,4-b]furan-8-yl)ethyl]propionamide, N-[2-(3,7,8,9-tetrahydropyrano[3,2-e]indol-1-yl)ethyl]propionamide, N-[2-(5-fluoro-3,7,8,9-tetrahydrocyclopenta[f][1]benzopyran-9-yl)ethyl]propionamide, N-[2-(3,7,8,9-tetrahydropyrano[3,2-e]indol-1-yl)ethyl]butylamide, N-[2-(1,2,3,7,8,9-hexahydropyrano[3,2-e]indol-1-yl)ethyl]propionamide, N-[2-(1,2,3,7,8,9-hexahydropyrano[3,2-e]indol-1-yl)ethyl]butylamide, N-[2-(4-fluoro-1,6,7,8-tetrahydro-2H-indeno[5,4-b]furan-8-yl)ethyl]butylamide, N-[2-(4-fluoro-1,6,7,8-tetrahydro-2H-indeno[5,4-b]furan-8-yl)ethyl]propionamide, (S)-N-[2-(1,6,7,8-tetrahydro-2H-indeno[5,4-b]furan-8-yl)ethyl]propionamide, (R)-N-[2-(1,6,7,8-tetrahydro-2H-indeno[5,4-b]furan-8-yl)ethyl]propionamide, N-[2-(1,6,7,8-tetrahydro-2H-indeno[5,4-b]furan-8-yl)ethyl]butylamide, N-[2-(1,6-dihydro-2H-indeno[5,4-b]furan-8-yl)ethyl]acetamide, N-[2-(1,6-dihydro-2H-indeno[5,4-b]furan-8-yl)ethyl]propionamide, N-[2-(1,6-dihydro-2H-indeno[5,4-b]furan-8-yl)ethyl]butylamide, N-[2-(7,8-dihydro-6H-indeno[4,5-d]-1,3-dioxol-8-yl)ethyl]propionamide, N-[2-(7,8-dihydro-6H-indeno[4,5-d]-1,3-dioxol-8-yl)ethyl]butylamide, N-[2-(2,3,8,9-tetrahydro-7H-indeno[4,5-b]-1,4-dioxyn-9-yl)ethyl]propionamide, N-[2-(2,3,8,9-tetrahydro-7H-indeno[4,5-b]-1,4-dioxyn-9-yl)ethyl]butylamide, N-[2-(1,6,7,8-tetrahydro-2H-furo[3,2-e]indol-8-yl)ethyl]propionamide, N-[2-(1,6,7,8-tetrahydro-2H-furo[3,2-e]indol-8-yl)ethyl]butylamide, N-[2-(7-phenyl-1,6-dihydro-2H-indeno[5,4-b]furan-8-yl)ethyl]propionamide, and N-[2-(7-phenyl-1,6-dihydro-2H-indeno[5,4-b]furan-8-yl)ethyl]butylamide.

More preferred are N-[2-(1,6,7,8-tetrahydro-2H-indeno[5,4-b]furan-8-yl)ethyl]acetamide, N-[2-(1,6,7,8-tetrahydro-2H-indeno[5,4-b]furan-8-yl)ethyl]propionamide, N-[2-(5-fluoro-3,7,8,9-tetrahydrocyclopenta[f][1]-benzopyran-9-yl)ethyl]propionamide, N-[2-(5-fluoro-1,2,3,7,8,9-hexahydrocyclopenta[f][1]benzopyran-9-yl)ethyl]propionamide, (S)-N-[2-(1,6,7,8-tetrahydro-2H-indeno[5,4-b]furan-8-yl)ethyl]propionamide, (R)-N-[2-(1,6,7,8-tetrahydro-2H-indeno[5,4-b]furan-8-yl)ethyl]propionamide, N-[2-(1,6,7,8-tetrahydro-2H-indeno[5,4-b]furan-8-yl)ethyl]butylamide, N-[2-(1,6-dihydro-2H-indeno[5,4-b]furan-8-yl)ethyl]acetamide, N-[2-(1,6-dihydro-2H-indeno[5,4-b]furan-8-yl)ethyl]propionamide, N-[2-(1,6-dihydro-2H-indeno[5,4-b]furan-8-yl)ethyl]butylamide, N-[2-(1,6,7,8-tetrahydro-2H-furo[3,2-e]indol-8-yl)ethyl]propionamide, N-[2-(1,6,7,8-tetrahydro-2H-furo[3,2-e]indol-8-yl)ethyl]butylamide, N-[2-(7-phenyl-1,6-dihydro-2H-indeno[5,4-b]furan-8-yl)ethyl]propionamide, and N-[2-(7-phenyl-1,6-dihydro-2H-indeno[5,4-b]furan-8-yl)ethyl]butylamide.

Especially preferred are (S)-N-[2-(1,6,7,8-tetrahydro-2H-indeno[5,4-b]furan-8-yl)ethyl]propionamide, N-[2-(1,6,7,8-tetrahydro-2H-furo[3,2-e]indol-8-yl)ethyl]propionamide, N-[2-(1,6,7,8-tetrahydro-2H-furo[3,2-e]indol-8-yl)ethyl]butylamide, N-[2-(7-phenyl-1,6-dihydro-2H-indeno[5,4-b]furan-8-yl)ethyl]propionamide, N-[2-(7-phenyl-1,6-dihydro-2H-indeno[5,4-b]furan-8-yl)ethyl]butylamide, and N-[2-(1,6,7,8-tetrahydro-2H-indeno[5,4-b]furan-8-yl)ethyl]acetamide.

Especially preferred compound (I) is the compound represented by the formula: 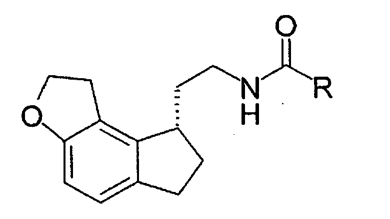 wherein R is C₁₋₆ alkyl group (methyl, ethyl, propyl, isopropyl, butyl, isobutyl, sec-butyl, ter-butyl, pentyl, hexyl, etc.); and concretely, (S)-N-[2-(1,6,7,8-tetrahydro-2H-indeno[5,4-b]furan-8-yl)ethyl]propionamide or (S)-N-[2-(1,6,7,8-tetrahydro-2H-indeno[5,4-b]furan-8-yl)ethyl]acetamide is preferred.

Salts of the compound (I) of the present invention include, for example, pharmaceutically acceptable salts thereof. For example, mentioned are salts with inorganic bases, salts with organic bases, salts with inorganic acids, salts with organic acids, salts with basic or acidic amino acids. Preferred examples of salts with inorganic bases include, for example, alkali metal salts such as sodium salts and potassium salts, alkaline earth metal salts such as calcium salts and magnesium salts,as well as aluminium salts and ammonium salts. Preferred examples of salts with organic bases include, for example, salts with trimethylamine, triethylamine, pyridine, picoline, 2,6-lutidine, ethanolamine, diethanolamine, triethanolamine, cyclohexylamine, dicyclohexylamine and N,N'-dibenzylethylenediamine. Preferred examples of salts with inorganic acids include, for example, salts with hydrochloric acid, hydrobromic acid, nitric acid, sulfuric acid and phosphoric acid. Preferred examples of salts with organic acids include, for example, salts with formic acid, acetic acid, trifluoroacetic acid, phthalic acid, fumaric acid, oxalic acid, tartaric acid, maleic acid, citric acid, succinic acid, malic acid, methanesulfonic acid, benzenesulfonic acid or p-toluenesulfonic acid. Preferred examples of salts with basic amino acids include, for example, salts with arginine, lysine and ornithine. Preferred examples of salts with acidic amino acids include, for example, salts with aspartic acid and glutamic acid.

Among others, preferred are pharmaceutically acceptable salts which include, for example, salts with inorganic acids such as hydrochloric acid, hydrobromic acid, nitric acid, sulfuric acid and phosphoric acid, and salts with organic acids such as acetic acid, phthalic acid, fumaric acid, tartaric acid, maleic acid, citric acid, succinic acid, methanesulfonic acid and p-toluenesulfonic acid, when the compound (I) has basic functional groups; or alkali metal salts such as sodium salts and potassium salts, and alkaline earth metal salts such as calcium salts and magnesium salts, and ammonium salts, when the compound (I) has acidic functional groups.

Compound (I) of the present invention may be hydrated or non-hydrated.

Compound (I) can be obtained in accordance with, for example, a process disclosed in Japanese Patent No. 2884153 and like processes.

Physicochemical properties of compounds having a melatonin receptor agonist activity suited for the percutaneous absorption preparations of the present invention include: moderate solubility to water (about 0.005 to about 10 mg/L) which allows the compound to be released from the formulation at an appropriate speed to distribute in the skin and finally absorbed in the general circulation, and partition ratio to oil (water/octanol partition coefficient: about 0.05 to about 10,000).

As for other conditions:
(1) those having a property that the compound does not become unstable in formulation;
(2) those having a property of not reacting with adhesive agents, skin permeation promoting agents used in formulation or generally used additives;
(3) those having a property of dissolving in about 0.1% by weight or more in volatile solvents such as alcohol, acetone, ethyl acetate and the likewhich are generally used in production of formulation;
(4) those having a molecular weight of not more than about 1000; and
(5) those having a melting point of not more than about 300 °C are preferred.

The percutaneous absorption preparation of the present invention can be produced by processes generally used for producing percutaneous absorption preparations and like processes .

As for the form for the percutaneous absorption preparations of the present invention, it is preferred to use, for example, those providing excellent handling, adherence to skin, and percutaneous absorptivity by sealing bandage treatment method, and concretely, those in which a so-called adhesive agent having adherence at ordinary temperatures is a base of a skin contact member, a plaster (skin plaster) in which an adhesive agent layer is formed on one side of a support member (backing layer) in view of the handling and the like.

In such a percutaneous absorption preparation, the compound having a melatonin receptor agonist activity serving as an active ingredient is preferably held by a skin contact member. Furthermore, while the skin contact member and the support member (backing layer) are made into one piece, the side not being in contact with the support member (backing layer) of the skin contact member may be protected by a protecting member such as release coated liner, or by making itself into a roll shape.

Furthermore, the skin contact member may not have cohesiveness. In such a case, the formulation is fixed by, for example, a tape and the like, thereby keeping the skin contact member and the skin in contact with each other.

The skin contact member is preferably principally made up of a compound having a melatonin receptor agonist activity which is an effective component, an adhesive agent and a skin permeation promoting agent. Furthermore, as is necessary, stabilizers, drug solubilizing agents, antibacterial agents, fillers, etc. may be contained.

It is preferred that the adhesive agent is made up of pharmaceutical adhesive agents, such as conventionally used (meth)acrylic adhesive agents, rubber type adhesive agents, and silicone type adhesive agents which have cohesiveness at ordinary temperatures and will not cause a rash and the like by insuring keratin when it comes into contact with the skin surface. Among these, (meth)acrylic adhesive agents which will not cause a chemical reaction, are stable in quality and superior in air permeability and cohesiveness are most preferred.

As the (meth)acrylic adhesive agent, a self-crosslinking type (meth)acrylic copolymer containing soft segments and hard segments is used. For example, a copolymer obtained by polymerization of an about 50 to 80% by weight of (meth)acrylic acid ester and an about 20 to 50% by weight of one or two kinds of copolymerizable monomers is used. As such a (meth)acrylic acid ester, an ester obtained from acrylic acid or methacrylic acid, and a primary to tertiary alcohol having 2 to 18, preferably 4 to 12 carbon atoms can be used.

Concrete (meth)acrylic adhesive agents include a copolymer composed of 2-hexyl acrylate and acrylic acid, a copolymer composed of 2-ethylhexyl acrylate and hydroxyethyl acrylate, a copolymer composed of 2-ethylhexyl acrylate and vinylpyrrolidone, a copolymer composed of 2-ethylhexyl acrylate and 2-methoxyethyl acrylate, a copolymer composed of 2-ethylhexyl acrylate and vinylpyrrolidone and acrylic acid, and the like.

As the rubber type adhesive agents, natural rubber, synthetic isoprene rubber, polyisobutylene, polyvinylether, polyurethane, polybutadiene, styrene-butadiene copolymer and the like are used.

As the silicone type adhesive agents, silicone rubbers such as polyorganosiloxane are used.

On the other hand, as the copolymerizable monomers, monomers having at least one unsaturated double bond involving the copolymerization reaction in the molecule, as well as having a functional group such as hydroxyl group, carboxyl group, amide group or amino group for its side chain can be used.

Examples of monomers having a hydroxyl group for its side chain include 2-hydroxyethyl (meth)acrylate, hydroxypropyl (meth)acrylate and the like.

Examples of monomers having a carboxyl group for its side chain include α-β unsaturated carboxylic acids such as (meth)acrylic acid, maleic acid monoalkylesters such as butyl maleate, maleic acid, fumaric acid, crotonic acid and the like.

Examples of the monomers having an amide group for its side chain include alkyl (meth)acrylamides such as acrylamide, dimethyl acrylamide and diethyl acrylamide, alkyl ethers of methylol (meth)acrylamide such as butoxymethyl acrylamide and ethoxymethyl acrylamide, diacetone acrylamide, vinyl pyrrolidone and the like.

Examples of monomers having an amino group for its side chain include dimethylamino acrylate and the like.

Examples of monomer that can polymerize other than the above include (meth)acrylonitrile, vinyl acetate, vinyl propionate, N-vinyl-2-pyrrolidone, methylvinylpyrrolidone, vinylpyridine, vinylpyperidone, vinylpyrimidine, vinylpiperazine, vinylpyrazine, vinylpyrrole, vinylimidazole, vinylcaprolactam, vinyloxazole, vinylformoline and the like.

As the copolymerizable monomers, monomers having at least one unsaturated double bond involving copolymerization reaction in its molecule, as well as having a hydroxyl group which is a functional group for its side chain are preferred. Examples of which include hydroxyethylmetacrylate (HEMA), hydroxypropylmetaacrylate (HPMA) and the like.

The polymerizing monomers as described above may copolymerized by one or more kinds of monomers, however, from the view points of adhesiveness in the meaning of the cohesiveness property and releasability of the compound having melatonin receptor agonist activity included in the skin contact member, those including at least one of the carboxylic group-containing monomer and hydroxyl group-containing monomer as an essential component are preferred. Furthermore, these monomers are used for copolymerization with (meth)acrylic acid ester in the range of about 1 to about 50% by weight, preferably about 3 to about 20% by weight. If necessary, the above- exemplified other monomers, for example, vinyl monomers such as vinyl acetate and N-vinyl-2-pyrrolidone can be copolymerized with (meth)acrylic acid in the range of not more than about 40% by weight, preferably not more than about 30% by weight.

The copolymers based on (meth)acrylic acid ester as described above are usually prepared by mixing the above-mentioned monomers in the presence of a polymerization primer and conducting solution polymerization. The solution polymerization can be conducted by adding ethyl acetate or other polymerization solvent to predetermined amounts of various monomers, and allowing the resultant mixture to react in a reactor equipped with a stirrer and a reflux condenser, in the presence of a polymerization initiator of azobis type or peroxide type, under the nitrogen atmosphere, at the temperature of about 70 to about 90 °C for about 8 to about 40 hours. The monomer may be introduced either by single loading or separated loading.

It is preferred that the ratio of the (meth)acrylic acid ester in the constituents of the copolymer based on the (meth)acrylic ester is about 50% by weight or more.

Examples of the above-mentioned azobis type polymerization initiator include 2,2-azobis-isobutyronitrile, 1,1'-azobis(cyclohexane-1-carbonitrile), 2,2'-azobis(2,4-dimethylvalerinitorile) and the like.

Examples of the above-mentioned peroxide type polymerization initiator include lauroyl peroxide, benzoyl peroxide, di(tert-butyl)peroxide and the like.

As the rubber type adhesive agents, natural rubber, synthetic isoprene rubber, polyisobutylene, polyvinylether, polyurethane, polybutadiene, styrene-butadiene copolymer, styrene-isoprene copolymer and the like are used.

As the silicone type adhesive agent, silicone rubbers such as polyorganosiloxane are used.

The skin permeation promoting agent is an agent which mainly acts on keratin which is the surface of the skin to facilitate permeation of the drug through the skin, thereby enabling efficient percutaneous absorption.

Generally, keratin is formed by plural layers of cell membranes overlapped with one after another, each cell membrane consisting of lipid bilayer generated as a result of metabolism of surface cells. Owing to this, harmful substances are prevented from easily entering the body.
This is also the reason why drugs are difficult to be absorbed percutaneously in the manner usually used.
Therefore, the main target of the skin permeation promoting agent is a lipid bilayer.

As the substance that acts on a lipid bilayer, strong surfactants such as detergent, solvents such as chloroform, ethers, benzenes and the like can be considered, however, these are not preferable because they stimulate and break a lipid bilayer, leading harmful actions.

Preferable properties of the skin permeation promoting agent include:
(1) improving fluidity of the membranes of a lipid bilayer;
(2) spreading a clearance of the layer structure of membrane by moisturizing the same;
(3) improving solubility of the compound having a melatonin receptor agonist activity in the skin contact member, to thereby increase the release speed from the formulation.

As the promoting agent that satisfies these properties and has a drug release characteristic that gives a one-peak blood-drug-concentration-time profile similar to the melatonin secretion pattern, the following (A), (B), (C) and the like can be exemplified. The percutaneous absorption preparation of the present invention contains three types of promoting agents, (A), (B) and (C).
(A) A lipid soluble absorption promoting agent which is a fatty acid ester composed of a fatty acid having 6 to 22 carbon atoms and an alcohol having 1 to 12 carbon atoms, and the like.
(B) A water soluble absorption promoting agent, which is a polyhydric alcohol.
(C) A nonionic surfactant. More preferably, fatty acid amides and the like such as lauric diethanolamide and compounds containing the same.

Examples of the above-mentioned fatty acids having 6 to 22 carbons include those having 6 to 22 carbons (for example, 10 to 22 carbons, more preferably 10 to 20 carbons) such as caproic acid, enanthic acid, caprylic acid, monocapric acid, oleic acid, lauric acid, undecylenic acid, myristic acid, isostearic acid, linoleic acid, palmitic acid, margaric acid, stearic acid, hexadecenoic acid, and the like.

Examples of the above-mentioned alcohols having 1 to 12 carbon atoms include methyl alcohol, ethyl alcohol, propanol, isopropanol, butanol, t-butanol, hexanol, octanol and the like. The "fatty acid" used in this context means natural or synthetically obtainable fatty acids in the same range as described above.

Therefore, examples of the above-mentioned fatty acid esters include isopropyl adipate, isopropyl myristate, diester sebacate, isopropyl palmitate, isopropyl stearate, butyl stearate, octyldodecyl myristate, hexyl laurate, octyl palmitate, ethyl oleate, butyl myristate and the like. Among these, isopropyl myristate, diester sebacate, isopropyl palmitate, butyl myristate and the like are preferred, and isopropyl myristate is particularly preferred.

Examples of the above-mentioned polyhydric alcohols include ethylene glycols (ethylene glycol, diethylene glycol, triethylene glycol), low molecular glycols such as glycerin, propyleneglycol and 1,3-butyleneglycol, high molecular glycols having a molecular weight of about 200 to about 6,000 such as polyethyleneglycol and polypropylene glycol, and the like, and among these ethylene glycols, propyleneglycol, 1,3-butyleneglycol, glycerin, polyethyleneglycol and the like are preferred, and propylene glycol and polyethylene glycol (molecular weight of about 200 to about 1000) are particularly preferred.

As the nonionic surfactant, for example, polyoxyethylene fatty acid esters, polyoxyethylene fatty acid esters, polyoxyethylene glyceryl fatty acid esters, polyoxyethylene alkyl ethers, polyoxyethylene alkylaryl ethers, glyceryl monooleate, glyceryl monolaurate, glyceryl monostearate, sorbitan monomyristate, sorbitan monopalmitate, sorbitan monooleate, polyoxyethylene (5) sorbitan monooleate, polyoxyethylene (20) sorbitan monooleate, derivatives of polyoxyethylene castor oil, block polymer type nonionic surfactants (e.g., pluronic, L-62, L-64, F-68, etc.), polyhydric alcohol fatty acid esters (e.g., glyceryl monooleate, glyceryl monolaurate, glyceryl monostearate, glyceryl monomyristate, glyceryl monopalmitate, glyceryl dioleate, glyceryl dilaurate, glyceryl distearate, glyceryl dimyristate, glyceryl dipalmitate, propylene glycol monocaprylate, caprylic / capric triglyceride, etc.), fatty acid esters of polyglycerin (for example, fatty acid esters of triglycerin (e.g., triglyceryl oleate, triglyceryl laurate, triglyceryl stearate, tryglyceryl myristate, triglyceryl palmitate), fatty acid esters of tetraglycerin (e.g., tetraglyceryl oleate, tetraglyceryl laurate, tetraglyceryl stearate, tetraglyceryl myristate, tetraglyceryl palmitate), fatty acid esters of pentaglycerin (e.g., pentaglyceryl oleate, pentaglyceryl laurate, pentaglyceryl stearate, pentaglyceryl myristate, pentaglyceryl palmitate), fatty acid esters of hexaglycerin (e.g., hexaglyceryl oleate, hexaglyceryl laurate, hexaglyceryl stearate, hexaglyceryl myristate, hexaglyceryl palmitate), fatty acid esters of heptaglycerin (e.g., heptaglyceryl oleate, heptaglyceryl laurate, heptaglyceryl stearate, heptaglyceryl myristate, heptaglyceryl palmitate), fatty acid esters of decaglycerin (e.g., decaglyceryl oleate, decaglyceryl laurate, decaglyceryl stearate, decaglyceryl myristate, decaglyceryl palmitate), and the like), fatty acid amides (oleic diethanolamide, myristic diethanolamide, stearic diethanolaminoethylamide, vinylpyrrolidone, lauric diethanolamide or substances containing the same, coconut fatty acid diethanolamide and the like), stearic diethylaminoethylamide, stearic dimethylaminopropylamide, lauric derivative quaternary ammonium salt, benzalkonium chloride aqueous solution, and the like) can be exemplified. Among these, fatty acid amides, fatty acid esters of polyhydric alcohol, fatty acid esters of polyglycerin are preferred, and in particular, fatty acid amides such as lauric diethanolamide or substances containing the same (skin permeation promoting agent containing the same) and coconut fatty acid diethanolamide are further preferred.

If required, antioxidants, a filler, a drug solubilizing agent, an antibacterial agent, a skin stimulation reducing agent, etc. may be added to the preparation of the present invention in addition to the above mentioned additives.

As the above antioxidant, vitamin E, vitamin C and the like can be exemplified.

As the above filler, kaolin, bentonite, titanium dioxide, silicon dioxide and the like can be exemplified.

As the above drug solubilizing agent, α-cyclodextrin, β-cyclodextrin, γ-cyclodextrin and the like can be exemplified.

As the above antibacterial agent, benzalkonium chloride, benzoic acid, metyl-p-hydroxybenzoate and the like can be exemplified.

As the skin stimulation reducing agent, silicic anhydride can be exemplified.

In addition, other absorption promoting agents can be added. As the other absorption promoting agents, polyprenylazacycloalkanes (for example, 1-dodecylazacycloheptane-2-on and the like), oils and fats (for example, olive oil, castor oil, jojoba oil, corn embryo oil, sunflower oil, coconut oil, squalane, squalene, orange oil, mineral oil) can be exemplified.

The skin permeation promoting agent comprises a fatty acid ester, a polyhydric alcohol and a nonionic surfactant. A preferred fatty acid ester is isopropyl myristate, isopropyl palmitate, butyl myristate or diethyl sebacate. And a preferred polyhydric alcohol is ethylene glycol, propylene glycol, 1,3-butylene glycol, glycerin or polyethylene glycol. A most preferred polyhydric alcohol is propylene glycol or polyethylene glycol. Particularly, it is preferred to blend silicon dioxide serving as a filler together with polyethylene glycol having a molecular weight of about 200 to about 1000, because the "stringiness(stickiness)" of adhesive agent is improved. Furthermore, a preferred nonionic surfactant is a fatty acid amide, a fatty acid ester of polyhydric alcohol or a fatty acid ester of polyglycerin. A most preferred nonionic surfactant is a fatty acid amide. A preferred fatty acid amide of that time is lauric diethanolamide or substances containing the same.

A most preferred fatty acid amide is lauric diethanolamide.

The formulation of the present invention improves the solubility in the skin contact member of the compound having a melatonin receptor agonist activity, and thus satisfies the releasability from the formulation.

When blending a compound having a melatonin receptor agonist activity in a skin contact member, it is preferred that the compound is blended in such a proportion that the action of the skin permeation promoting agent is fully spread out, facilitating permeation of the compound having a melatonin receptor agonist activity.

For example,
(1) A content of a compound having a melatonin receptor agonist activity with respect to the whole skin contact member is about 0.01 to about 70% by weight, more preferably about 10 to about 60% by weight, and further preferably about 20 to about 50% by weight;
(2) A content of a skin permeation promoting agent with respect to the whole skin contact member is about 0 to about 70% by weight, more preferably about 10 to about 60% by weight, and further preferably about 20 to about 50% by weight;
   For the skin permeation promoting agent individual weights of the fatty acid ester, the polyhydric alcohol and the nonionic surfactant in the skin contact member are, about 0 to about 70% by weight, and preferably about 1 to about 30% by weight (about 1 to about 15% by weight is preferred for the nonionic surfactant). Furthermore, the blend proportion of the polyhydric alcohol is about 1/10 to about 10 times in weight, more preferably about 1/2 to about 5 times in weight, most preferably about once in weight of that of the fatty acid ester based on the blend weight of the fatty acid ester. Furthermore, the blend proportion of the nonionic surfactant is about 1/50 to about 10 times in weight, more preferably about 1/20 to about 2 times in weight, and most preferably about 1/4 of that of the fatty acid ester
(3) A content of adhesive agent with respect to the whole skin contact member is about 5 to about 98% by weight, preferably about 10 to about 60% by weight, and more preferably about 20 to about 50% by weight;

Materials such as anti-oxidant, filler, drug solubilizing agent, antibacterial agent as described above can be blended in a skin contact member as other ingredients as is necessary. These components are added within the range that will not deteriorate the adhesiveness of the skin contact member and the effect of the skin permeation promoting agent, and the amount of blend thereof is about 0.01 to about 50% by weight, preferably about 1 to about 20% by weight, more preferably about 1 to about 10% by weight.

A plaster which is one embodiment of the formulation of the present invention can be obtained by pasting a support member (backing layer) on one surface of the adhesive agent layer and a release liner on the other surface of the adhesive agent layer.

As the support member (backing layer) of the plaster, any materials can be available insofar as they have an effect of preventing water volatilization and moisturizing a skin which are necessary to allowing the active ingredient in the formulation according to the present invention to be absorbed efficiently after administration, and they enable patients to easily affix the present formulation on their skins and will not give abnormal feeling even after a long time of affixing. For example, a film formed of polyethylene, polypropylene, cellulose acetate, ethyl cellulose, polyethylene terephthalate, vinyl acetate-vinyl chloride copolymer, plastic poly(vinyl chloride), polyurethane, polyolefin or poly(vinylidene chloride) or an aluminum foil having a thickness of about 50 to about 200 µm can be exemplified. These may be used in the form of a single layer sheet (film) or a lamination sheet, and woven or nonwoven fabric using materials other than aluminum foil can also be used.

As for the release liner, since the release liner is used as a "cover" for preventing the active ingredient in the present percutaneous absorption formulation from coming into contact with other object to pollute the same, or from being scraped to be impaired before use, any material is available insofar as a patient can easily remove it when using the present formulation and the skin contact member after removal of the release liner still keeps the condition before being covered with the release liner. For example, siliconized polyethylene terephthalate film, paper, polyester, low density polyethylene, high density polyethylene, polypropylene, polystyrene, polyamide, nylon, polyvinyl chloride and the like having a thickness of 50 to about 100 µm can be used.

The skin contact member can be formed by dissolving a composition containing an adhesive agent, a skin permeation promoting agent and a compound melatonin receptor agonist activity in an appropriate solvent, applying the resultant adhesive-containing solution on a supporting member (backing layer), and removing the solvent by drying.

As a manufacturing method of a plaster which is one embodiment of the formulation according to the present invention, a method in which a skin contact member is applied on a supporting member and a release liner is pasted on the surface of the skin contact member, and a method in which a skin contact member is applied on a release liner and a supporting member is pasted on the surface of the skin contact member can be exemplified. For application of the skin contact member, a solution in which a composition of a skin contact member is dissolved or a dispersed solution in which a part of the composition is dispersed is prepared by adding a variety of skin permeation promoting agents into a high concentration solution of the adhesive agent dissolved in an easily volatile solvent dispersion solution and mixing them well, and adding the compound having melatonin receptor agonist activity of the present invention and mixing them well. As an easily volatile solvent which preferred in this case, those easily vaporize under appropriated dry condition (typically, the condition of heating for 1 hour at 50°C or the condition of placing at room temperature for all day and night) and will not remain in the skin contact member which is a final product or will not be harmful on a living body even if a small amount remains are selected. For example, mixture solutions in which about 0 to about 500% by weight of isopropyl alcohol or acetone is contained in ethyl alcohol or ethyl acetate can be used.

It is preferred that the concentration of the adhesive agent in the solvent is high for the purpose of improving the application efficiency, however, too high concentration is not preferred for achieving uniform application. Concentration for use is in the range of about 10% by weight to about 500% by weight and preferably about 20% by weight to about 150% by weight. Concentrations in solvent of constituents of skin contact member other than the adhesive agent are automatically determined when the blend proportions with respect to the adhesive are determined. Since it is preferred that the compound having a melatonin receptor agonist activity is dissolved as much as possible, a method in which of the compound is previously dissolved in an easily volatile solvent at high concentration and then added as a solvent solution is preferably applied. Examples of the preferred easily volatile solvent include the solvents used for dissolving the above-mentioned adhesive agent which will not remain in the skin contact member after drying, acetone, ethyl alcohol, methyl alcohol and the like. Acetone or ethyl acetate is preferred. Concentration of the compound having a melatonin receptor agonist activity in the solvent is selected to be supersaturation or concentrations nearly supersaturation. As such a concentration, about 1 to about 20% by weight is used. In the case where the amount of blend of the compound having a melatonin receptor agonist activity is large, a part of the compound will not dissolve. However, also in this case, since it is preferred that the individual particles are microparticles, powder of the compound having a melatonin receptor agonist activity is grained well before dissolving it in the solvent.

As the application method, a method including: fixing a supporting member (backing layer) or a release liner on a uniform plate such as glass plate; dropping a solution of a composition of a skin contact member in solvent thereon; spreading the solution by means of a roller such as a commercially available applicator (casting device) (Baker Applicator; Yoshimitsu Seiki) in such a condition that the solvent is spread into a uniform thickness; and thereafter placing it at room temperature for all day and night to evaporate the solvent. As the evaporating condition, heating for 30 minutes at 50°C in the initial stage may be used because it makes it possible to rapidly evaporate the solvent. The method as described above is a method for applying a relatively small amount, however, rotary continuous manufacturing machine that have been improved for mass production and generally used can be used. The thickness obtainable by dropping the solution in solvent of the composition of the skin contact member and spreading the same by means of a roller in such a condition that leads a uniform thickness is determined to be larger than the thickness of the skin contact member in contemplation of the volume of the solvent that is inversely calculated from the concentration. The thickness of the skin contact member is in the range of about 0.01 mm to about 5 mm, preferably about 0.05 mm to about 1 mm.

The formulation according to the present invention can be cut into pieces of appropriate size that can achieve the object prior to use.

The blend amount of the compound having a melatonin receptor agonist activity in the formulation of the present invention is not particularly limited insofar as the compound is absorbed into the blood from the skin after administration, the blood concentration of the active ingredient is less than the concentration that leads a side effect, and the effective concentration can be kept for a long time. The blend amount of the compound having a melatonin receptor agonist activity is, for example, about 0.1 to about 60% by weight, preferably about 0.1 to about 20% by weight, more preferably about 1 to about 10% by weight of the total weight of the formulation. In the case where the formulation of the present invention is a plaster, blend amount of the compound having a melatonin receptor agonist activity per unit area of the skin contact region is, for example, about 0.01 to about 100 mg/cm², preferably about 1 to about 100 mg/cm², more preferably about 2 to about 50 mg/cm², further preferably about 5 to about 10 mg/cm². Typical effective concentration of the compound having a melatonin receptor agonist activity which is less than the concentration that leads a side effect is about 0.5 to about 1,000 ng/mL, more particularly about 1 to about 500 ng/mL.

Administration (affix) frequency for the formulation of the present invention is, for example, once every 1 to 7 days, preferably once every 1 to 3 days, more preferably once a day. Administration period for the formulation of the present invention is usually one month to five years, and may be administered for a longer period so as to prevent development of the symptom. The administration period is preferably 3 months to four years, more preferably 6 months to two years. During such long period administration, the formulation of the present invention can be readily administered without putting a load on a patient.

In the case where the formulation of the present invention is a patch or a tape, the formulation may be cut into a convenient size and one or more pieces may be affixed on the same site or different sites on the body.
The site to affix the formulation is not particularly limited, however, sites with little body hair are preferable and, for example, the formulation is affixed to the arm region inside, back, femoral region inside, and the like. Among these, the arm region is preferred.

It is preferable for a blood concentration pattern of a compound having a melatonin receptor agonist activity to resemble a secretion pattern of melatonin of a normal person. That is, as reported in Journal of Clinical Endocrinology and Metabolism 73: 1276-1280 (1991), melatonin secretion of a normal person rises in the night, and the melatonin concentration in the blood represents a one-peak pattern from the evening to the morning. Therefore, it is desirable for blood-drug-concentration-time-profile to draw a one-peak pattern from the evening to the morning (within 12 hours after administration).

In this case, a preferred timing of administration of the absorption agent is in the evening or before going to bed (between 6 hours before bedtime or just before bedtime).

It is preferred for the peak of the blood concentration to appear in about 10 hours after administration.

It is preferable for the effective concentration of the compound to be maintained until about one to two hours before getting up and be damped afterwards. A duration time of effective concentration corresponds to a sleep time, and is preferably about 6 to about 12 hours.

The formulation of the present invention is useful for a pharmaceutical product because it has low toxicity and causes little side effect.

Dosage of the formulation of the present invention varies according to the type and content of the compound having a melatonin receptor agonist activity which is a principal component, dosage form, duration time of release of the compound having a melatonin receptor agonist activity, objective disease, objective animal and the like, however, it can be an effective amount of the compound having the melatonin receptor agonist activity. A single dosage of the compound having a melatonin receptor agonist activity which is a principal component can be selected appropriately from, for example, the range of about 0.05 mg to 10 mg/kg body weight per adult person, preferably from a range of about 0.1 mg to 3mg/kg body weight per adult person.

The formulation of the present invention acts as a melatonin agonist or antagonist for mammals (for example, mouse, rat, hamster, rabbit, cat, dog, cow, sheep, monkey, human); and is useful as a melatonin receptor affinity composition, in particular, as a composition having a melatonin receptor agonist activity; and can be used for prevention and treatment of sleep-awake rhythm disorder, jet lag (jetlag), abnormality of physical condition by three change duty, severe depression of a season, genital and neuroendocrine disease, senile dementia, Alzheimer's disease, various disorders associated with aging (for example, antiaging), cerebral circulation disorder (for example, cerebral stroke), head injury, marrow damage, stress, epilepsia, cramp, uneasiness, depression, Parkinson's disease, high blood pressure, glaucoma, cancer, insomnia, diabetes and the like; and is also effective for immunoregulation, enhancement of cognition, ataractic or ovulation adjustment (for example, sterilization). The formulation of the present invention is used, for example, as a biological rhythm adjustment agent, preferably a therapeutic agent for somnipathy (for example, sleep leading agent and the like), sleep-awake rhythm adjustment agent (including sleep-awake rhythm adjusting action), and a prevention and treatment agent for time zone change syndrome, a so-called jet lag (jetlag). For instance, in the case of treatment of a somnipathist, a formulation of the present invention containing an about 1 to about 10% by weight of an active ingredient is applied on inside of the arm once a day for one month.

Furthermore, the formulation of the present invention may be used, as appropriate, in combination with an appropriate amount of other active agents other than the compound having a melatonin receptor agonist activity (for example, benzodiazepinic drugs such as triazolam, diazepam, alprazolam, estazolam which are benzodiazepine compounds, non-benzodiazepinic drugs such as zolpidem, zalepron, zopiclone, brotizoram and the like, sleep rhythm adjustment agents such as butoctamide which is a fatty acid derivative or its salt, hypnotics such as cis-9,10-octadecenoamide).

In the following , the present invention will be further explained while referring to test examples and comparative examples.

### Examples

### Example 1

**[Table 1]**

| Composition of skin contact member | Percentage with respect to adhesive layer |
|---|---|
| (Adhesive agent) | |
| Self-crosslinking acrylic copolymer | 47.5% |

| (Skin permeation promoting agent) | |
|---|---|
| Lauric diethanolamide | 5.0% |
| Isopropyl myristate | 20.0% |
| Propyleneglycol | 20.0% |

| (Active ingredient) | |
|---|---|
| Compound A | 7.5% |

To a solution of 45% (w/w) of self-crosslinking acrylic copolymer (DuroTak^{™} 87-2979; National Starch & Chemical) in 8:2 (ratio in volume) ethyl acetate/isopropanol, lauric diethanolamide (AMINONE^{™} L-02; KAO Corporation Chemicals), isopropyl myristate, propyleneglycol and (S)-N-[2-(1,6,7,8-tetrahydro-2H-indeno[5,4-b]furan-8-yl)ethyl]propionamide (referred to as Compound A) were added in the respective blend ratios of 5.0% by weight, 20.0% by weight and 7.5% by weight of the total weight of the skin contact member and mixed well, and 5.6 g of this mixture solution was dropped on a fluoropolymer-treated polyester film (Scotchpak^{™} 1022; product of 3M, thickness:75 µm, total area including a merge part:450 cm²) spread on a plate of a casting device (Baker applicator: Yoshimitsu Seiki), and spread so that the thickness after drying will be 0.1 mm by means of a roller. Thereafter the solvent was air dried at room temperature for all day and night, and a support member (backing layer, polyethylene film, CoTran^{™} 9720; product of 3M, thickness:76µm) was pasted on the surface opposite to the skin contact surface, thereby obtaining a percutaneous absorption preparation of the present invention.

### Example 2

**[Table 2]**

| Composition of skin contact member | Percentage with respect to adhesive layer |
|---|---|
| (Adhesive agent) | |
| Self-crosslinking acrylic copolymer | 53.0% |

| (Skin permeation promoting agent) | |
|---|---|
| Lauric diethanolamide | 5.0% |
| Isopropyl myristate | 20.0% |
| Propyleneglycol | 20.0% |

| (Active ingredient) | |
|---|---|
| Compound A | 2.0% |

To a solution of 45% (w/w) of self-crosslinking acrylic copolymer (DuroTak^{™} 87-2979; National Starch & Chemical) in 8:2 (ratio in volume) ethyl acetate/isopropanol, lauric diethanolamide (AMINONE^{™} L-02; KAO Corporation Chemicals), isopropyl myristate, propyleneglycol and Compound A were added in the respective blend ratios of 5.0% by weight, 20.0% by weight, 20.0% by weight and 2.0% by weight of the total weight of the skin contact member and mixed well, and a percutaneous absorption preparation of the present invention was obtained in the same condition and manner as Example 1.

### Example 3

A composition in which a self-crosslinking acrylic copolymer which is an adhesive agent, lauric diethanolamide and Compound A which is an active ingredient are mixed in the proportion of 93:5:2 (w/w) was prepared, and a percutaneous absorption preparation of the present invention was obtained in the same condition and manner as Example 1.

### Comparative Example 1

**[Table 3]**

| | A | B | C |
|---|---|---|---|
| Composition of skin contact member | Percentage with respect to adhesive layer | | |
| (Adhesive agent) | | | |
| Self-crosslinking acrylic copolymer | 58.0 | 73.0 | 73.0 |

| (Skin permeation promoting agent) | | | |
|---|---|---|---|
| Lauric diethanolamide | 0.0 | 5.0 | 5.0 |
| Isopropyl myristate | 20.0 | 0.0 | 20.0 |
| Propyleneglycol | 20.0 | 20.0 | 0.0 |

| (Active ingredient) | | | |
|---|---|---|---|
| Compound A | 2.0 | 2.0 | 2.0 |

As shown in [Table 3], skin contact member compositions of three prescriptions (Rp. A to C) each excluding one of the three kinds of skin permeation promoting agents in Example 2 were prepared, and percutaneous absorption preparations of the present invention were prepared in the same manner as Example 1. Example 4

A percutaneous absorption preparation of the present invention was prepared in the same manner as Example 1 in such a composition that in place of propyleneglycol in Example 1, the same amount of 1,3-butyleneglycol is blended.

### Example 5

A percutaneous absorption preparation of the present invention was prepared in the same manner as Example 1 in such a composition that in place of propyleneglycol in Example 1, the same amount of polyethyleneglycol having a molecular weight of 400 is blended.

### Example 6

A percutaneous absorption preparation of the present invention was prepared in the same manner as Example 1 in such a composition that in place of isopropyl myristate in
Example 1, the same amount of isopropyl palmitate is blended.

### Example 7

A percutaneous absorption preparation of the present invention was prepared in the same manner as Example 1 in such a composition that in place of isopropyl myristate in Example 1, the same amount of butyl myristate is blended.

### Example 8

A percutaneous absorption preparation of the present invention was prepared in the same manner as Example 2 in such a composition that in place of isopropyl myristate in Example 2, the same amount of diethyl sebacate is blended.

### Example 9

A percutaneous absorption preparation of the present invention was prepared in the same manner as Example 2 in such a composition that in place of Compound A in Example 1, the same amount of N-[2-(1,6,7,8-tetrahydro-2H-indeno[5,4-b]furan-8-yl)ethyl]acetamide is blended.

### Example 10

A percutaneous absorption preparation of the present invention was prepared in the same manner as Example 1 in such a composition that in place of lauric diethanolamine in Example 1, the same amount of coconut fatty acid diethanol amide is blended.

### Example 11

**[Table 4]**

| Composition of skin contact member | Percentage with respect to adhesive layer |
|---|---|
| (Adhesive agent) | |
| Self-crosslinking acrylic copolymer | 68.0% |

| (Skin permeation promoting agent) | |
|---|---|
| Lauric diethanolamide | 10.0% |
| Isopropyl myristate | 10.0% |
| Polyethyleneglycol 600 | 10.0% |

| (Filler) | |
|---|---|
| Silicon dioxide | 20.0% |

| (Active ingredient) | |
|---|---|
| Compound A | 2.0% |

To a solution of 41% (w/w) of self-crosslinking acrylic copolymer (DuroTak^{™}387-2516; National Starch & Chemical) in 8:2 (ratio in volume) ethyl acetate/isopropanol, lauric diethanolamide (AMINONE^{™} L-02; KAO Corporation Chemicals), isopropyl myristate, polyethyleneglycol 600, silicon dioxide and Compound A were added in the respective blend ratios of 10.0% by weight, 10.0% by weight, 10.0% by weight, 20.0% by weight and 2.0% by weight of the total weight of the skin contact member (120%) and mixed well, and a percutaneous absorption preparation of the present invention was obtained in the same condition and manner as Example 1.

### Example 12

A percutaneous absorption preparation of the present invention was prepared in the same manner as Example 1 in such a composition that in place of Compound A in Example 11, the same amount of (S)-N-[2-(1,6,7,8-tetrahydro-2H-indeno[5,4-b]furan-8-yl)ethyl]acetamide obtained in Reference example 1 is blended.

### Reference example 1

### (S)-N-[2-(1,6,7,8-tetrahydro-2H-indeno[5,4-b]furan-8-yl)ethyl]acetamide

To a solution of (S)-2-[1,6,7,8-tetrahydro-2H-indeno[5,4-b]furan-8-yl]ethylamine hydrochloride (71.92 g, 0.3 mol) in dichloromethane (500 mL), triethylamine (104.6 mL, 0.75 mol), dimethylaminopyridine (3.67 g, 0.03 mol) and acetic anhydride (31.2 mL, 0.33 mol) were added under icecooling, and stirred four 16 hour at room temperature. The reaction mixture was poured into cold water and the organic layer was separated. The organic layer was washed with 1N hydrochloric acid and saturated brine and dried over sodium sulfate, followed by purification by a small amount of silica gel chromatography (dichloromethane). After distilling off the solvent under reduced pressure, the obtained crystal was recrystalized from isopropyl ether/ethyl acetate to give the title compound (yield: 53.2 g, 72%).
Melting point: 118-120°C
NMR(CDCl₃) δ: 1.50-1.92(2H, m), 1.96(3H,s), 1.96-2.13(1H,m), 2.19-2.38(1H,m), 2.67-2.95(2H,m), 3.00-3.9(5H,m), 4.43-4.64(2H,m), 5.43(1H,br), 6.62(1H,d,J=7.8Hz), 6.95(1H,d,J=7.8Hz).
Elemental Analysis for C₁₅H₁₉NO₂
Calcd: C,73.44, H,7.81, N,5.71
Found: C,73.56, H,7.89, N5.86
Angle of rotation: [α]_{D}=-59.1°(c=1.0%, chloroform)

### Test example 1

Male SD rats in 7 weeks-old (body weight about 250 g, 3 or 4 per one administration group) were anaesthetized by ether, and after shaving the body hair of abdomen, percutaneous absorption preparations according to Examples 1 and 2 which are cut into pieces so that the affix area becomes 30 cm² or 7.1 cm² were affixed, and the pieces were wounded and fixed by stretchable bandage from above so that the plaster will not come off. The contents of Compound A in the administered percutaneous absorption preparations were calculated to be 27 mg and 9 mg per 30 cm², respectively.

After affixing, the rats were placed back to the respective cages under no anesthesia, and blood samples were collected at regular interval from tail veins, and the blood concentrations of the Compound A were quantified by means of the HPLC.

### 1) Extraction of drug from plasma

0.1 mL of plasma was taken in a 10 mL test tube, to which 0.5 mL of 0.05 M phosphoric buffer (pH7) and 5 mL of diethylether were added. After shaking for 15 minutes, the drug was extracted by ether, and 4.5 mL of the ether solution was evaporated and dried to be solidified and then dissolved by adding an HPLC eluate to give an HPLC quantification sample.

### 2) HPLC condition

Column: TSKgel ODS-80Ts QA (4.6 mmI.D., 150 mm, Tosoh)
Eluate 1: 0.01 M CH₃COONH₄/CH₃CN (ratio in volume 60:40)
Eluate 2: 0.01 M CH₃COONH₄/CH₃CN (ratio in volume 10:90)
Flow rate: 1 mL/min
Gradient program:

| | | | | | |
|---|---|---|---|---|---|
| Time (min) | 0 | 7 | 12 | 15 | 15.1 |
| Eluate 1 | 100% | 100% | 0% | 0% | 100% |
| Eluate 2 | 0% | 0% | 100% | 100% | 0% |

Column temperature: 40°C
Detection: at UV 210 nm
Maximum concentration of Compound A in plasma after affixing each administration (Cmax) and its reach time (Tmax) and bioavailability (BA) of the same formulation for intravenous administration will be shown in Table 5.

**[Table 5]**

| Administered sample | Affix area | Cmax | Tmax | BA |
|---|---|---|---|---|
| Percutaneous absorption preparation of Example 1 | 30 cm² | 2200 ng/mL | 8 hours | 61% |
| | 7.1 cm² | 250 ng/mL | 8 hours | 40% |
| Percutaneous absorption preparation of Example 2 | 30 cm² | 580 ng/mL | 6 hours | 52% |
| | 7.1 cm² | 200 ng/mL | 6 hours | 52% |

In the percutaneous absorption preparations of Examples 1 and 2, one peak of blood-drug-concentration-time profile that reaches the maximum blood concentration 6 to 8 hours after affixing was observed, and it was found that the amount of absorption relies upon loading amount and affixing area. In addition, no administration groups show any abnormality in the post-experimental observation of the skin part where the agent had been affixed conducted.

### Test example 2

The percutaneous absorption preparation of Example 3 was administered to rats by affixing the preparation on their abdomens in the same manner as Test example 1 and blood concentration of Compound A after administration was measured in the same manner as Test example 1. Average plasma level during 0 to 24 hours was about 50 ng/mL and BA was about 12%.

### Comparative Test example 1

The percutaneous absorption preparation of Comparative Example 1 was administered to rats by affixing the preparation on their abdomen in the same manner as Test example 1 and blood concentration of Compound A after administration was measured in the same manner as Test example 1. As for Prescriptions A and B, average plasma concentration during 0 to 24 hours was about 50 ng/mL and BA of each prescription was 15% and 20%, respectively. Prescription C showed Cmax 210 ng/mL at 8 hours, and BA of 30%.

### Comparative example 2

A composition in which a self-crosslinking acrylic copolymer which is an adhesive agent and Compound A which is an active ingredient were blended in a proportion of 98:2 (w/w) was prepared, and a comparative percutaneous absorption preparation was obtained in the same condition and manner as Example 1. The obtained agent was administered to rats by affixing the agent on their abdomens in the same manner as Test example 1 and blood concentration of Compound A after administration was measured in the same manner as Test example 1. Average plasma level during 0 to 24 hours was not more than 10 ng/mL, and a blood-drug-concentration-time profile not having a clear Cmax was observed, and BA was about 2%.

From the above, it can be concluded that the percutaneous absorption preparations of the present invention enable the active ingredient to be absorbed into the body through a skin contact surface by a convenient administration system, providing a favorable blood-drug-concentration-time profile in which the blood concentration of the active ingredient is kept for 6 to 12 hours.

### Industrial Applicability

The percutaneous absorption preparations of the present invention enable a compound having a melatonin receptor agonist activity to be absorbed by a convenient administration system, present favorable blood-drug-concentration-time profile in which blood concentration of the active ingredient is kept for 6 to 12 hours in contrast to the case of oral administration, and can exert an therapeutic effect on a disease caused by a decrease in melatonin secretion at night.

## Claims

1. A percutaneous absorption preparation containing a compound having a melatonin receptor agonist activity represented by the formula: 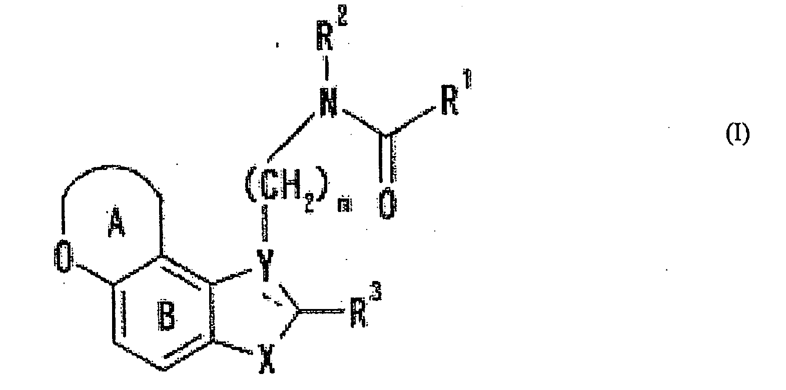 wherein, R¹ represents an optionally substituted hydrocarbon group, an optionally substituted amino group or an optionally substituted heterocyclic group;
R² represents a hydrogen atom or an optionally substituted hydrocarbon group;
R³ represents a hydrogen atom, an optionally substituted hydrocarbon group or an optionally substituted heterocyclic group;
X represents CHR⁴, NR⁴, O or S in which R⁴ represents a hydrogen atom or an optionally substituted hydrocarbon group;
Y represents C, CH or N, provided that when X is CH₂, Y is C or CH;
-̅-̅-̅-̅-̅ represents a single bond or a double bond;
ring A represents an optionally substituted, 5- to 7- membered oxygen-containing heterocyclic ring;
ring B represents an optionally substituted benzene ring; and
m represents an integer of 1 to 4;
or a salt thereof,
and a fatty acid ester, a polyhydric alcohol and a non-ionic surfactant.

2. The percutaneous absorption preparation according to claim 1, wherein the compound having a melatonin receptor agonist activity is a compound having a melatonin ML₁ receptor agonist activity.

3. The percutaneous absorption preparation according to claim 1, wherein the compound having a melatonin receptor agonist activity is a compound represented by the formula: 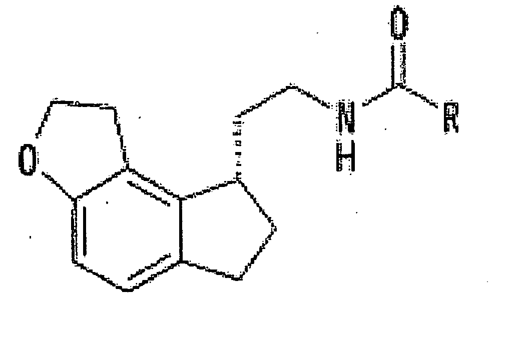 wherein, R represents a C₁₋₆ alkyl group.

4. The percutaneous absorption preparation according to claim 1, wherein the compound having a melatonin receptor agonist activity is (S)-N-[2-(1,6,7,8-tetrahydro-2H-indeno[5,4-b]furan-8-yl)ethyl]propionamide.

5. The percutaneous absorption preparation according to claim 1, wherein the compound having a melatonin receptor agonist activity is (S)-N-[2-(1,6,7,8-tetrahydro-2H-indeno[5,4-b]furan-8-yl)ethyl]acetamide.

6. The percutaneous absorption preparation according to claim 1, wherein the fatty acid ester is an ester of a carboxylic acid having 6 to 22 carbon atoms and an alkyl alcohol having 1 to 12 carbon atoms.

7. The percutaneous absorption preparation according to claim 1, wherein the fatty acid ester is isopropyl myristate, isopropyl palmitate, butyl myristate, or diethyl sebacate.

8. The percutaneous absorption preparation according to claim 1, wherein the fatty acid ester is isopropyl myristate.

9. The percutaneous absorption preparation according to claim 1, wherein the polyhydric alcohol is ethylene glycol, propylene glycol, 1,3-butylene glycol, glycerin or polyethylene glycol.

10. The percutaneous absorption preparation according to claim 1, wherein the polyhydric alcohol is propylene glycol.

11. The percutaneous absorption preparation according to claim 1, wherein the polyhydric alcohol is polyethylene glycol.

12. The percutaneous absorption preparation according to claim 1, wherein the polyhydric alcohol is polyethylene glycol having a molecular weight of about 200 to about 1000.

13. The percutaneous absorption preparation according to claim 1, wherein the nonionic surfactant is a fatty acid amide, a polyhydric alcohol fatty acid ester or a polyglycerol fatty acid ester.

14. The percutaneous absorption preparation according to claim 1, wherein the nonionic surfactant is a fatty acid amide.

15. The percutaneous absorption preparation according to claim 14, wherein the fatty acid amide is lauric diethanol amide or a compound including the same.

16. The percutaneous absorption preparation according to claim 14; wherein the fatty acid amide is coconut fatty acid diethanol amide.

17. The percutaneous absorption preparation according to claim 1 containing (S)-N-[2-(1,6,7,8-tetrahydro-2H-indeno[5,4-b]furan-8-yl)ethyl]propionamide, isopropyl myristate, polyethylene glycol and lauric diethanol amide.

18. The percutaneous absorption preparation according to claim 1 containing (S)-N-[2-(1,6,7,8-tetrahydro-2H-indeno[5,4-b]furan-8-yl)ethyl]acetamide, isopropyl myristate, polyethylene glycol and lauric diethanol amide.

19. The percutaneous absorption preparation according to claim 1 which is a skin plaster.

20. The percutaneous absorption preparation according to claim 1 containing in a skin contact member, a compound having a melatonin receptor agonist activity and a fatty acid ester, a polyhydric alcohol and a nonionic surfactant.

21. The percutaneous absorption preparation according to claim 20 containing in a skin contact member, an about 1 to about 30% by weight of fatty acid ester with respect to a weight of the skin contact member.

22. The percutaneous absorption preparation according to claim 20 containing in a skin contact member, an about 1 to about 30% by weight of polyhydric alcohol with respect to a weight of the skin contact member.

23. The percutaneous absorption preparation according to claim 20 containing in a skin contact member, an about 1 to about 15% by weight of nonionic surfactant with respect to a weight of the skin contact member.

24. The percutaneous absorption preparation according to claim 20 containing in a skin contact member, an adhesive agent.

25. The percutaneous absorption preparation according to claim 24, wherein the adhesive agent is an acrylic adhesive agent.

26. The percutaneous absorption preparation according to claim 20 containing in a skin contact member, an about 0.01 to about 70% by weight of compound having a melatonin receptor agonist activity with respect to a weight of the skin contact member.

27. The percutaneous absorption preparation according to claim 20 containing in a skin contact member, an about 5 to about 99% by weight of adhesive agent with respect to a weight of the skin contact member.

28. The percutaneous absorption preparation according to claim 20, wherein a content of the compound having a melatonin receptor agonist activity per unit skin contact surface of a skin contact member is about 0.01 to about 100 mg/cm².

29. The percutaneous absorption preparation according to claim 20 containing in a skin contact member, a filler.

30. The percutaneous absorption preparation according to claim 29, wherein the filler is silicon dioxide.

31. The percutaneous absorption preparation according to claim 1 which is to be affixed between about 6 hours before bedtime to just before bedtime.

32. The percutaneous absorption preparation according to claim 30, wherein a peak of the effective blood concentration of the compound having a melatonin receptor agonist activity appears within about 10 hours after administration.

33. Use of a compound having a melatonin receptor agonist activity represented by the formula: 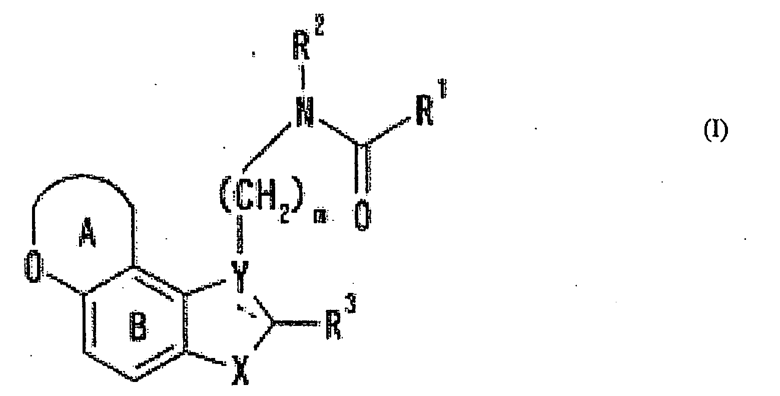 wherein, R¹ represents an optionally substituted hydrocarbon group, an optionally substituted amino group or an optionally substituted heterocyclic group;
R² represents a hydrogen atom or an optionally substituted hydrocarbon group;
R³ represents a hydrogen atom, an optionally substituted hydrocarbon group or an optionally substituted heterocyclic group;
X represents CHR⁴, NR⁴, O or S in which R⁴ represents a hydrogen atom or an optionally substituted hydrocarbon group;
Y represents C, CH or N, provided that when X is CH₂, Y is C or CH;
-̅-̅-̅-̅-̅ represents a single bond or a double bond;
ring A represents an optionally substituted, 5- to 7- membered oxygen-containing heterocyclic ring;
ring B represents an optionally substituted benzene ring; and
m represents an integer of 1 to 4;
or a salt thereof, and a fatty acid ester, a polyhydric alcohol and a nonionic surfactant in the manufacture of a percutaneous absorption medicament for the preventive and therapeutic treatment of diseases related to melatonin.

## Patentansprüche

1. Präparat für perkutane Resorption, enthaltend eine Verbindung, die eine Melatonin-Rezeptor-agonistische Aktivität aufweist und durch die Formel 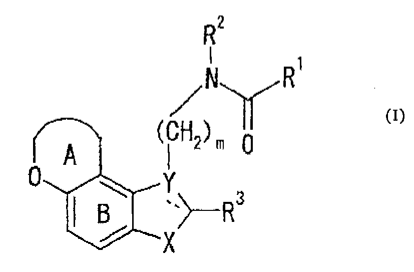 dargestellt wird, wobei R¹ für eine gegebenenfalls substituierte Kohlenwasserstoffgruppe, eine gegebenenfalls substituierte Aminogruppe oder eine gegebenenfalls substituierte heterocyclische Gruppe steht;
R² für ein Wasserstoffatom oder eine gegebenenfalls substituierte Kohlenwasserstoffgruppe steht;
R³ für ein Wasserstoffatom, eine gegebenenfalls substituierte Kohlenwasserstoffgruppe oder eine gegebenenfalls substituierte heterocyclische Gruppe steht;
X für CHR⁴, NR⁴, O oder S steht, wobei R⁴ für ein Wasserstoffatom oder eine gegebenenfalls substituierte Kohlenwasserstoffgruppe steht;
Y für C, CH oder N steht, mit der Maßgabe, dass Y = C oder CH ist, wenn X = CH₂ ist;
-̅-̅-̅-̅-̅ für eine Einfachbindung oder eine Doppelbindung steht;
Ring A für einen gegebenenfalls substituierten fünf- bis siebengliedrigen sauerstoffhaltigen heterocyclischen Ring steht;
Ring B für einen gegebenenfalls substituierten Benzolring steht; und
m für eine ganze Zahl von 1 bis 4 steht;
oder ein Salz davon sowie einen Fettsäureester, einen mehrwertigen Alkohol und ein nichtionisches Tensid.

2. Präparat für perkutane Resorption gemäß Anspruch 1, wobei die Verbindung, die eine Melatonin-Rezeptor-agonistische Aktivität aufweist, eine Verbindung ist, die eine Melatonin-ML₁-Rezeptor-agonistische Aktivität aufweist.

3. Präparat für perkutane Resorption gemäß Anspruch 1, wobei die Verbindung, die eine Melatonln-Rezeptor-agonistische Aktivität aufweist, eine Verbindung ist, die durch die Formel 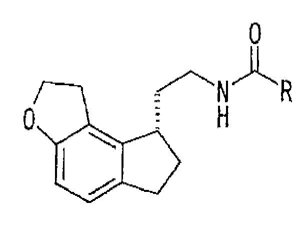 dargestellt wird, wobei R für eine C₁₋₆-Alkylgruppe steht.

4. Präparat für perkutane Resorption gemäß Anspruch 1, wobei es sich bei der Verbindung, die eine Melatonin-Rezeptor-agonistische Aktivität aufweist, um (S)-N-[2-(1,6,7,8-Tetrahydro-2H-indeno[5,4-b]furan-8-yl)ethyl]propionamid handelt.

5. Präparat für perkutane Resorption gemäß Anspruch 1, wobei es sich bei der Verbindung, die eine Melatonin-Rezeptor-agonistische Aktivität aufweist, um (S)-N-[2-(1,6,7,8-Tetrahydro-2H-indeno[5,4-b]furan-8-yl)ethyl]acetamid handelt.

6. Präparat für perkutane Resorption gemäß Anspruch 1, wobei der Fettsäureester ein Ester einer Carbonsäure mit 6 bis 22 Kohlenstoffatomen und eines Alkylalkohols mit 1 bis 12 Kohlenstoffatomen ist.

7. Präparat für perkutane Resorption gemäß Anspruch 1, wobei es sich bei dem Fettsäureester um Isopropylmyristat, Isopropylpalmitat, Butylmyristat oder Diethylsebacat handelt.

8. Präparat für perkutane Resorption gemäß Anspruch 1, wobei es sich bei dem Fettsäureester um Isopropylmyristat handelt.

9. Präparat für perkutane Resorption gemäß Anspruch 1, wobei es sich bei dem mehrwertigen Alkohol um Ethylenglycol, Propylenglycol, 1,3-Butylenglycol, Glycerin oder Polyethylenglycol handelt.

10. Präparat für perkutane Resorption gemäß Anspruch 1, wobei es sich bei dem mehrwertigen Alkohol um Propylenglycol handelt.

11. Präparat für perkutane Resorption gemäß Anspruch 1, wobei es sich bei dem mehrwertigen Alkohol um Polyethylenglycol handelt.

12. Präparat für perkutane Resorption gemäß Anspruch 1, wobei es sich bei dem mehrwertigen Alkohol um Polyethylenglycol mit einem Molekulargewicht von etwa 200 bis etwa 1000 handelt.

13. Präparat für perkutane Resorption gemäß Anspruch 1, wobei das nichtionische Tensid ein Fettsäureamid, ein Fettsäureester eines mehrwertigen Alkohols oder ein Polyglycerinfettsäureester ist.

14. Präparat für perkutane Resorption gemäß Anspruch 1, wobei das nichtionische Tensid ein Fettsäureamid ist.

15. Präparat für perkutane Resorption gemäß Anspruch 14, wobei es sich bei dem Fettsäureamid um Laurinsäurediethanolamid oder eine Verbindung, die dieses umfasst, handelt.

16. Präparat für perkutane Resorption gemäß Anspruch 14, wobei es sich bei dem Fettsäureamid um Kokosnussfettsäurediethanolamid handelt.

17. Präparat für perkutane Resorption gemäß Anspruch 1, das (S)-N-[2-(1,6,7,8-Tetrahydro-2H-indeno[5,4-b]furan-8-yl)ethyl]propionarnid, Isopropylmyristat, Polyethylenglycol und Laurinsäurediethanolamid enthält.

18. Präparat für perkutane Resorption gemäß Anspruch 1, das (S)-N-[2-(1,6,7,8-Tetrahydro-2H-indeno[5,4-b]furan-8-yl)ethyl]acetamid, Isopropylmyristat, Polyethylenglycol und Laurinsäurediethanolamid enthält.

19. Präparat für perkutane Resorption gemäß Anspruch 1, bei dem es sich um ein Hautpflaster handelt.

20. Präparat für perkutane Resorption gemäß Anspruch 1, das ein Hautkontaktelement, einer Verbindung mit Melatonin-Rezeptor-agonistischer Aktivität sowie einen Fettsäureester, einen mehrwertigen Alkohol und ein nichtionisches Tensid enthält.

21. Präparat für perkutane Resorption gemäß Anspruch 20, das in einem Hautkontaktelement etwa 1 bis etwa 30 Gew.-% Fettsäurenester enthält, bezogen auf das Gewicht des Hautkontaktelements.

22. Präparat für perkutane Resorption gemäß Anspruch 20, das in einem Hautkontaktelement etwa 1 bis etwa 30 Gew.-% mehrwertigen Alkohol enthält, bezogen auf das Gewicht des Hautkontaktelements,

23. Präparat für perkutane Resorption gemäß Anspruch 20, das in einem Hautkontaktelement etwa 1 bis etwa 15 Gew.-% nichtionisches Tensid enthält, bezogen auf das Gewicht des Hautkontaktelements.

24. Präparat für perkutane Resorption gemäß Anspruch 20, das in einem Hautkontaktelement ein Klebemittel enthält.

25. Präparat für perkutane Resorption gemäß Anspruch 24, wobei das Klebemittel ein Acrylkleber ist.

26. Präparat für perkutane Resorption gemäß Anspruch 20, das in einem Hautkontaktelement etwa 0,01 bis etwa 70 Gew.-% einer Verbindung, die eine Melatonin-Rezeptor-agonistische Aktivität aufweist, enthält, bezogen auf das Gewicht des Hautkontaktelements.

27. Präparat für perkutane Resorption gemäß Anspruch 20, das in einem Hautkontaktelement etwa 5 bis etwa 99 Gew.-% Klebemittel enthält, bezogen auf das Gewicht des Hautkontaktelements.

28. Präparat für perkutane Resorption gemäß Anspruch 20, wobei der Gehalt an der Verbindung, die eine Melatonin-Rezeptor-agonistische Aktivität aufweist, pro Einheit der Hautkontaktfläche eines Hautkontaktelements etwa 0,01 bis etwa 100 mg/cm² beträgt.

29. Präparat für perkutane Resorption gemäß Anspruch 20, das in einem Hautkontaktelement einen Füllstoff enthält.

30. Präparat für perkutane Resorption gemäß Anspruch 29, wobei es sich bei dem Füllstoff um Siliciumdioxid handelt.

31. Präparat für perkutane Resorption gemäß Anspruch 1, das zwischen etwa 6 Stunden vor dem Schlafengehen bis unmittelbar vor dem Schlafengehen angeheftet werden soll.

32. Präparat für perkutane Resorption gemäß Anspruch 30, wobei ein Maximum der effektiven Blutkonzentration der Verbindung, die eine Melatonin-Rezeptor-agonistische Aktivität aufweist, innerhalb von etwa 10 Stunden nach der Verabreichung erscheint.

33. Verwendung einer Verbindung, die eine Melatonin-Rezeptor-agonistische Aktivität aufweist und durch die Formel 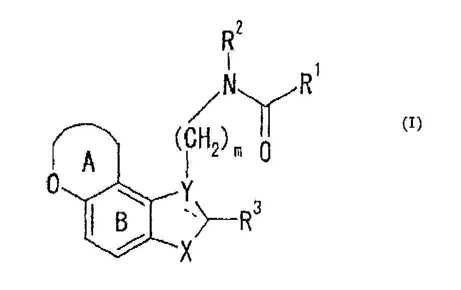 dargestellt wird, wobei R¹ für eine gegebenenfalls substituierte Kohlenwasserstoffgruppe, eine gegebenenfalls substituierte Aminogruppe oder eine gegebenenfalls substituierte heterocyclische Gruppe steht;
R² für ein Wasserstoffatom oder eine gegebenenfalls substituierte Kohlenwasserstoffgruppe steht;
R³ für ein Wasserstoffatom, eine gegebenenfalls substituierte Kohlenwasserstoffgruppe oder eine gegebenenfalls substituierte heterocyclische Gruppe steht;
X für CHR⁴, NR⁴, O oder S steht, wobei R⁴ für ein Wasserstoffatom oder eine gegebenenfalls substituierte Kohlenwasserstoffgruppe steht;
Y für C, CH oder N steht, mit der Maßgabe, dass Y = C oder CH ist, wenn X = CH₂ ist;
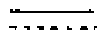 für eine Einfachbindung oder eine Doppelbindung steht;
Ring A für einen gegebenenfalls substituierten fünf- bis siebengliedrigen sauerstoffhaltigen heterocyclischen Ring steht;
Ring B für einen gegebenenfalls substituierten Benzolring steht; und
m für eine ganze Zahl von 1 bis 4 steht;
oder eines Salzes davon sowie eines Fettsäureesters, eines mehrwertigen Alkohols und eines nichtionischen Tensids bei der Herstellung eines Medikaments für perkutane Resorption für die präventive und therapeutische Behandlung von Krankheiten, die mit Melatonin zusammenhängen.

## Revendications

1. Préparation d'absorption percutanée contenant un composé ayant une activité agoniste des récepteurs à la mélatonine représenté par la formule : 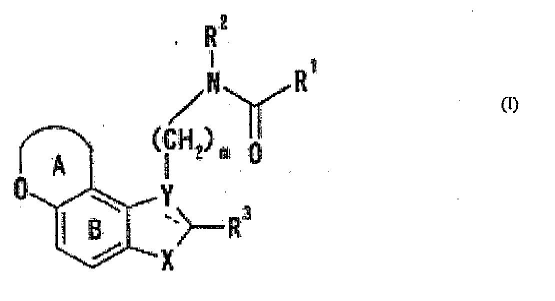 dans laquelle, R¹ représente un groupe hydrocarbure facultativement substitué, un groupe amino facultativement substitué ou un groupe hétérocyclique facultativement substitué ;
R² représente un atome d'hydrogène ou un groupe hydrocarbure facultativement substitué,
R³ représente un atome d'hydrogène, un groupe hydrocarbure facultativement substitué ou un groupe hétérocyclique facultativement substitué ;
X représente CHR⁴, NR⁴, O ou S dans lesquels R⁴ représente un atome d'hydrogène ou un groupe hydrocarbure facultativement substitué ;
Y représente C, CH ou N, à condition que lorsque X est CH₂, Y soit C ou CH ;
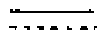 représente une liaison simple ou une liaison double ;
le cycle A représente un cycle hétérocyclique contenant un atome d'oxygène, à 5 à 7 chaînons et facultativement substitué ;
le cycle B représente un cycle benzène facultativement substitué ; et
m représente un nombre entier de 1 à 4 ;
ou un sel de celui-ci,
et un ester d'acide gras, un alcool polyhydrique et un tensioactif non ionique.

2. Préparation d'absorption percutanée selon 1a revendication 1, dans laquelle le composé ayant une activité agoniste des récepteurs à la mélatonine est un composé ayant une activité agoniste du récepteur à la mélatonine ML₁.

3. Préparation d'absorption percutanée selon 1a revendication 1, dans laquelle le composé ayant une activité agoniste des récepteurs à 1a mélatonine est un composé représenté par 1a formule : 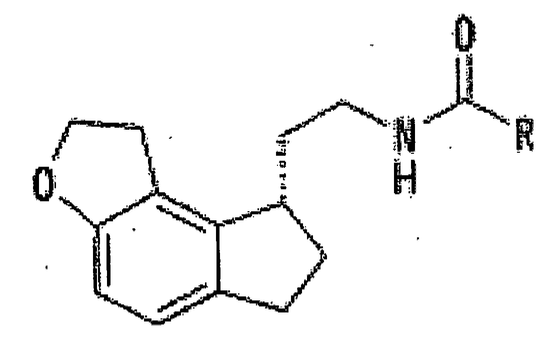 dans laquelle, R représente un groupe alkyle en C₁₋₆.

4. Préparation d'absorption percutanée selon la revendication 1, dans laquelle le composé ayant une activité agoniste des récepteurs à la mélatonine est le (S)-N-[2-(1,6,7,8-tétrahydro-2H-indéno[5,4-b]furan-8-yl)éthyl)propionamide.

5. Préparation d'absorption percutanée selon la revendication 1, dans laquelle le composé ayant une activité agoniste des récepteurs à la mélatonine est le (S)-N-[2-(1,6,7,8-tétrahydro-2H-indéno[5,4-b]furan-8-yl)éthyl)acétamide.

6. Préparation d'absorption percutanée selon la revendication 1, dans laquelle l'ester d'acide gras est un ester d'un acide carboxylique ayant de 5 à 22 atomes de carbone et d'un alcool alkylique ayant de 1 à 12 atomes de carbone.

7. Préparation d'absorption percutanée selon la revendication 1, dans laquelle l'ester d'acide gras est le myristate d'isopropyle, le palmitate d'isopropyle, le myristate de butyle, ou le sébacate de diéthyle.

8. Préparation d'absorption percutanée selon la revendication 1, dans laquelle l'ester d'acide gras est le myristate d'isopropyle.

9. Préparation d'absorption percutanée selon la revendication 1, dans laquelle l'alcool polyhydrique est l'éthylène glycol, le propylèneglycol, le 1,3-butylène glycol, la glycérine ou le polyéthylène glycol.

10. Préparation d'absorption percutanée selon la revendication 1, dans laquelle l'alcool polyhydrique est le propylène glycol.

11. Préparation d'absorption percutanée selon la revendication 1, dans laquelle l'alcool polyhydrique est le polyéthylène glycol.

12. Préparation d'absorption percutanée selon la revendication 1, dans laquelle l'alcool polyhydrique est le polyéthylène glycol ayant une masse moléculaire d'environ 200 à environ 1 000.

13. Préparation d'absorption percutanée selon la revendication 1, dans laquelle le surfactant non ionique est un amide d'acide gras, un ester d'acide gras et d'alcool polyhydrique ou un ester d'acide gras et de polyglycérol.

14. Préparation d'absorption percutanée selon la revendication 1, dans laquelle le surfactant non ionique est un amide d'acide gras.

15. Préparation d'absorption percutanée selon, la revendication 14, dans laquelle l'amide d'acide gras est un amide de diéthanol laurique ou un composé le comprenant.

16. Préparation d'absorption percutanée selon la revendication 14, dans laquelle l'amide d'acide gras est l'amide de diéthanol d'acide gras de coco.

17. Préparation d'absorption percutanée selon la revendication 1, qui contient du (S)-N-[2-(1,6,7,8-tétrahydro-2H-indéno[5,4-b]furan-8-yl)éthyl]propionamide, du myristate d'isopropy:le, du polyéthylène glycol et un amide de diéthanol laurique.

18. Préparation d'absorption percutanée selon la revendication 1, qui contient du (S)-N-[2-(1,6,7,8-tétrahydro-2H-indéno[5,4-b]furan-8-yl)éthyl]acétamide, du myristate d'isopropyle, du polyéthylène glycol et un amide de diéthanol laurique.

19. Préparation d'absorption percutanée selon la revendication 1, qui est un pansement pour la peau.

20. Préparation d'absorption percutanée selon la revendication 1, qui contient dans un élément destiné à être en contact avec la peau, un composé ayant une activité agoniste des récepteurs à la mélatonine et un ester d'acide gras, un alcool polyhydrique et un tensioactif non ionique.

21. Préparation d'absorption percutanée selon la revendication 20, qui contient dans un élément destiné à être en contact avec la peau, d'environ 1 à environ 30 % en poids d'un ester d'acide gras relativement à un poids de l'élément destiné à être en contact, avec la peau.

22. Préparation d'absorption percutanée selon la revendication 20, qui contient dans un élément destiné à être en contact avec la peau, d'environ 1 à environ 30 % en poids d'un alcool polyhydrique relativement à un poids de l'élément destiné à être en contact avec la peau.

23. Préparation d'absorption percutanée selon la revendication 20, qui contient dans un élément destiné à être en contact avec la peau, d'environ 1 à environ 15 % en poids d'un surfactant non ionique relativement à un poids de l'élément destiné à être en contact avec à peau.

24. Préparation d'absorption percutanée selon la revendication 20, qui contient dans un élément destine à être en contact avec la peau, un agent adhésif.

25. Préparation d'absorption percutanée selon la revendication 24, dans laquelle l'agent adhésif est un agent adhésif acrylique.

26. Préparation d'absorption percutanée selon la revendication 20, qui contient dans un élément destiné à être en contact avec la peau, d'environ 0,01 à environ 70 % en poids d'un composé ayant une activité agoniste des récepteurs à la mélatonine relativement à un poids de l' élément destiné à être en contact, avec la peau.

27. Préparation d'absorption percutanée selon la revendication 20, qui contient dans un élément destiné à être en contact avec la peau, d'environ 5 à environ 99 % en poids d'un agent adhésif relativement à un poids de l'élément destiné à être en contact avec la peau.

28. Préparation d'absorption percutanée selon la revendication 20, dans laquelle une teneur en le composé ayant une activité agoniste des récepteurs à la mélatonine par unité de surface de contact avec la peau d'un élément destiné à être en contact avec la peau est d'environ 0,01 environ 100 mg/cm².

29. Préparation d'absorption percutanée selon la revendication 20, qui contient dans un élément destiné à être en contact avec la peau, une charge.

30. Préparation d'absorption percutanée selon la revendication 29, dans laquelle la charge est le dioxyde de silicium.

31. Préparation d'absorption percutanée selon la revendication 1, qui est destinée à être appliquée d'environ 6 heures avant le coucher à juste avant le coucher.

32. Préparation d'absorption percutanée selon à revendication 30, dans laquelle un pic de la concentration efficace dans le sang du composé ayant une activité agoniste des récepteurs à la mélatonine apparaît dans les 10 heures suivant l'administration.

33. Utilisation d'un composé ayant une activité agoniste des récepteurs à la mélatonine représenté par la formule : 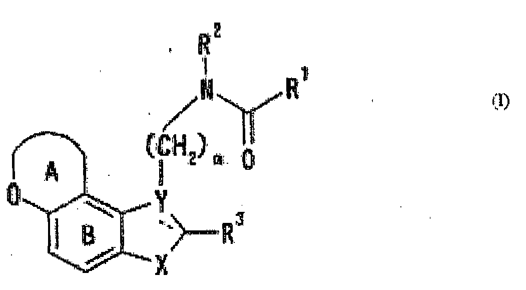 dans laquelle, R¹ représente un groupe hydrocarbure facultativement substitué, un groupe amino facultativement substitué ou un groupe hétérocyclique facultativement substitué ;
R² représente un atome d'hydrogène ou un groupe hydrocarbure facultativement substitué,
R³ représente un atome d'hydrogène, un groupe hydrocarbure facultativement substitué ou un groupe hétérocyclique facultativement substitué ;
X représente CHR₄, NR₄, O ou S dans lesquels R₄ représente un atome d'hydrogène ou un groupe hydrocarbure facultativement substitué ;
Y représente C, CH ou N, à condition que lorsque X est CH₂, Y soit C ou CH ;
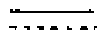 représente une liaison simple ou une liaison double ;
le cycle A représente un cycle hétérocyclique contenant un atome d'oxygène, à 5 à 7 chaînions et facultativement substitué ;
le cycle B représente un cycle benzène facultativement substitué ; et
m représente un nombre entier de 1 à 4 ;
ou un sel de celui-ci, et un ester d'acide gras, un alcool polyhydrique et un surfactant non ionique dans la fabrication d'un médicament d'absorption percutanée pour le traitement préventif et thérapeutique de maladies liées à la mélatonine.
